# EUROPÄISCHE PATENTSCHRIFT

(11) **EP 0 773 264 B1**
(45) Veröffentlichungstag und Bekanntmachung des Hinweises auf die Patenterteilung: **10.10.2001**
(21) Anmeldenummer: 96117605.4
(22) Anmeldetag: 04.11.1996
(51) Int. Cl.: C09B 62/04, C09B 62/503, C09B 62/20, C09B 62/002, C09B 19/02, C07D 498/04

(54) **Verfahren zur Herstellung von beidseitig amidierten Triphendioxazinfarbstoffen und neue Triphendioxazinfarbstoffe**
Process for the preparation of triphenodioxazine dyes amidated on both sides and triphenodioxazine dyes
Procédé pour la préparation de colorants triphènedioxazines amides des deux côtés et colorants triphènedioxazines

(30) Priorität: 10.11.1995 DE 19541985
(43) Veröffentlichungstag der Anmeldung: 14.05.1997
(73) Patentinhaber: DyStar Textilfarben GmbH & Co. Deutschland KG, 60318 Frankfurt am Main (DE)
(72) Erfinder: Schumacher, Christian, Dr., 65779 Kelkheim (DE); Herd, Karl-Josef, Dr., 51519 Odenthal (DE)

(56) Entgegenhaltungen:
- EP-A- 0 385 120
- EP-A- 0 628 606
- WO-A-94/21646
- FR-A- 2 095 580
- BULLETIN OF THE CHEMICAL SOCIETY OF JAPAN, Bd. 56, Nr. 5, Mai 1983, TOKYO JP, Seiten 1482-1486, XP002026132 H.NISHI ET AL.: "a new synthesis of triphenodithiazines and triphenodithiazinequinones"

## Beschreibung

Die vorliegende Erfindung liegt auf dem Gebiet der Triphendioxazin-Textilfarbstoffe.

Triphendioxazine sind farbstarke Farbstoffe, die sowohl als Direkt- als auch als Reaktivfarbstoffe geeignet sind. Die entsprechenden Farbstoffhydrolysate sind jedoch häufig schlecht auswaschbar, und die Egalität der Färbungen ist unbefriedigend.

Beidseitig amidierte Dioxazine wurden in der Fachliteratur schon häufig beschrieben (z.B. EP-A-0 275 022, EP-A-0 325 246, DE-A-2 124 080). Vielfach entstehen bei den beschriebenen Verfahrensweisen jedoch hauptsächlich die einseitig amidierten Verbindungen, oftmals im Gegensatz zu den gemachten Angaben, oder die als beidseitig amidiert beschriebenen Produkte entstehen nur in unbefriedigenden Ausbeuten.
So sind die einseitig amidierten Verbindungen königsblau, während die beidseitigen amidierten Verbindungen violett oder sehr rotstichtig blau sind. Es ist weiterhin bekannt, daß mit überschüssigen Amidierungsmitteln ein immer röteres Produkt entsteht (DE-A-2 124 080), was auf eine teilweise Doppelamidierung schließen läßt, die aber in den seltensten Fällen selektiv gelingt.

Weiterhin liefern bislang übliche Methoden zur Herstellung solcher Triphendioxazin-Farbstoffe, z.B. EP-A-0 325 246, EP-A-0 385 120 und EP-A-0 275 022, verbesserungsbedürftige Ausbeuten und Produktqualitäten.

Unter "amidierten Triphendioxazin-Verbindungen" sind in der vorliegenden Anmeldung solche Verbindungen gemeint, bei denen die exocyclischen Stickstoffatome des Triphendioxazinchromophors mit einem Acylrest, einem Aminocarbonylrest, einem Sulfonylrest oder einem stickstoffhaltigen heteroaromatischen Rest verbunden sind.

Aufgabe der vorliegenden Erfindung war es, ein Verfahren zur Herstellung von Triphendioxazin-Farbstoffen bereitzustellen, welches im Hinblick auf Ausbeute und Produktqualität die Nachteile des Standes der Technik überwindet.

Eine weitere Aufgabe der vorliegenden Erfindung war, eine vorteilhafte Methode zur Herstellung von beidseitig amidierten Triphendioxazinen zur Verfügung zu stellen.

Eine weitere Aufgabe der Erfindung war es, neue Triphendioxazin-Farbstoffe bereitzustellen, insbesondere solche mit violetter oder rotstichig blauer Nuance.

Es wurde gefunden, daß die genannten Aufgaben überraschenderweise durch Reduktion des gegebenenfalls bereits einseitig amidierten TriphendioxazinChromophors zur Leuko-Form, Amidierung auf der Stufe der Leuko-Form und Reoxidation gelöst werden.

Gegenstand der Erfindung ist ein Verfahren zur Herstellung von Triphendioxazin-Verbindungen der allgemeinen Formel (1) 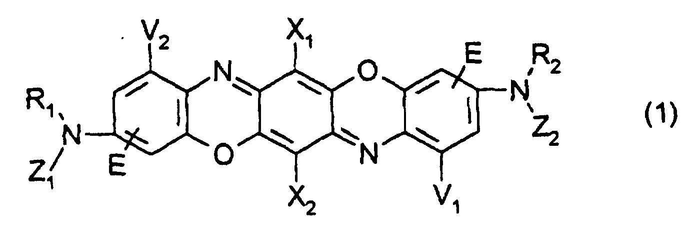 worin
- R₁: Wasserstoff oder C₁-C₄-Alkyl ist, das durch 1 bis 2 Substituenten aus der Reihe Hydroxy, C₁-C₄-Alkoxy, Sulfato oder Sulfo substituiert sein kann;
- R₂: eine der Bedeutungen von R₁ hat;
- E: Sulfo, Carboxy, C₁-C₄-Alkylsulfonyl oder ein Rest SO₂Y ist,
in welchem Y Vinyl oder CH₂CH₂V ist, worin V für Hydroxy oder für eine Abgangsgruppe aus der Reihe Sulfato, Phosphato, Thiosulfato oder Halogen, wie Chlor, steht;
oder -SO₂NR₃R₄ oder -CONR₃R₄ ist, worin
R₃ Wasserstoff, Phenyl, oder C₁-C₄-Alkyl ist, das durch Hydroxy, Carboxy, Sulfo, Sulfato oder einen Rest SO₂Y substituiert sein kann,
R₄ eine der Bedeutungen von R₃ hat, oder zusammen mit R₃ und N einen 5- oder 6-gliedigen Heterocyclus bildet, der durch 1 bis 3 weitere Heteroatome aus der Reihe N, O und S unterbrochen sein kann;
- X₁: Halogen, wie Chlor oder Brom, insbesondere Chlor, Wasserstoff, C₁-C₆-Alkyl, wie Methyl, Ethyl oder Isopropyl, Phenyl, Phenoxy oder C₁-C₄-Alkoxy ist;
- X₂: eine der Bedeutungen von X₁ hat;
- V₁: Wasserstoff, Sulfo, Methoxy, Methyl oder Halogen, wie Chlor, ist;
- V₂: eine der Bedeutungen von V₁ hat; und
- Z₁ und Z₂: gleich oder verschieden sind und einen Acylrest, einen unsubstituierten, alkylierten oder arylierten Aminocarbonylrest, einen Sulfonylrest oder einen stickstoffhaltigen heteroaromatischen Rest bedeuten,
dadurch gekennzeichnet, daß man eine Verbindung der Formel (2) 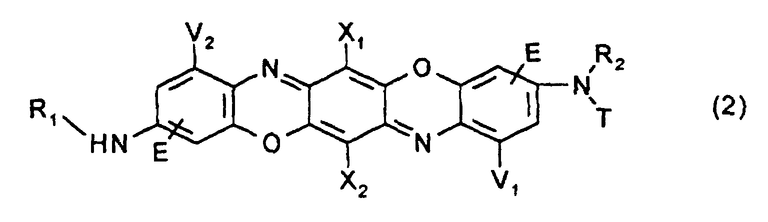 worin T Wasserstoff, Z₁ oder Z₂ ist,
zu einer Verbindung der Formel (3) reduziert, 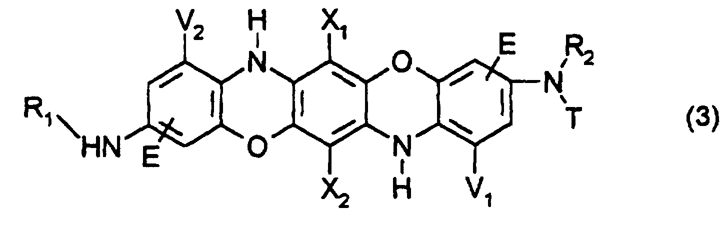 diese mit einem dem Rest Z₁ und/oder Z₂ zugrundeliegenden reaktiven Derivat zu einer Verbindung der Formel (4) umsetzt 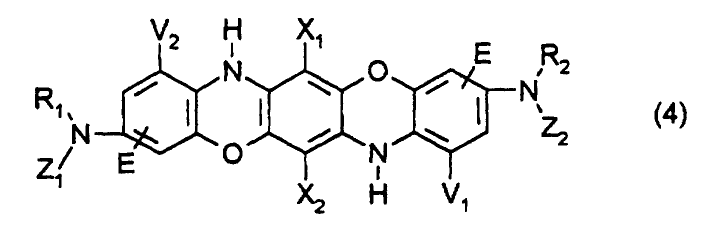 und diese anschließend zur Triphendioxazin-Verbindung der Formel (1) oxidiert.

Aus Bull. Chem. Soc. Jpn. 56 (1983), 1482 ist bekannt, daß 9,13-Dichlor-Triphendioxazine mit Zinn in Polyphosphorsäure in 9- und 13-Positionen dehalogeniert werden. Anstelle der nach der Kenntnis des Standes der Technik erwarteten Dehalogenierung findet jedoch überraschenderweise eine Reduktion zur Leukobase statt, wenn man die erfindungsgemäße Umsetzung im wäßrigen Medium in Gegenwart von Reduktionsmitteln durchführt. Die Reduktion ist vollständig reversibel, man erhält durch Oxidation wieder die Triphendioxazin-Farbbase.

Bevorzugt steht E für Sulfo, -SO₂-CH₂CH₂-OSO₃M, -SO₂-CH=CH₂,
-SO₂-CH₂CH₂Cl oder -SO₂-CH₂CH₂OH.
Bevorzugt stehen X₁ und X₂ für Chlor.
Bevorzugt steht V₁ für Wasserstoff.
Bevorzugt steht V₂ für Wasserstoff oder Sulfo, insbesondere für Wasserstoff.
Bevorzugt stehen R₁ und R₂ für Wasserstoff.
Bevorzugt ist R₃ Wasserstoff, Methyl oder Phenyl.
Bevorzugt stehen die Reste Z₁ und Z₂ für C₁-C₆-Alkyl-carbonyl, C₂-C₄-Alkenyl-carbonyl, C₆-Aryl-carbonyl, Aminocarbonyl, C₁-C₆-Alkylaminocarbonyl, Di-(C₁-C₆)-alkylaminocarbonyl, C₆-Arylamino-carbonyl oder C₆-Aryl-sulfonyl, wobei die Reste Alkyl und Aryl durch 1 bis 3, bevorzugt 1, gleiche oder verschiedene Substituenten aus der Reihe SO₂Y, Sulfo, Carboxy, Hydroxy, C₁-C₄-Alkoxy, C₁-C₄-Alkyl, Cyano, Halogen, Acylamino, wie Acetylamino, und Nitro substituiert sein können.

Für den Fall, daß Z₁ und/oder Z₂ einen stickstoffhaltigen heterocyclischen Rest bedeuten, so sind Heterocyclen aus der Reihe der Triazine, Pyrimidine und Chinoxaline, bevorzugt.
Insbesondere bevorzugt sind dabei Reste der allgemeinen Formeln (5a) bis (5d) 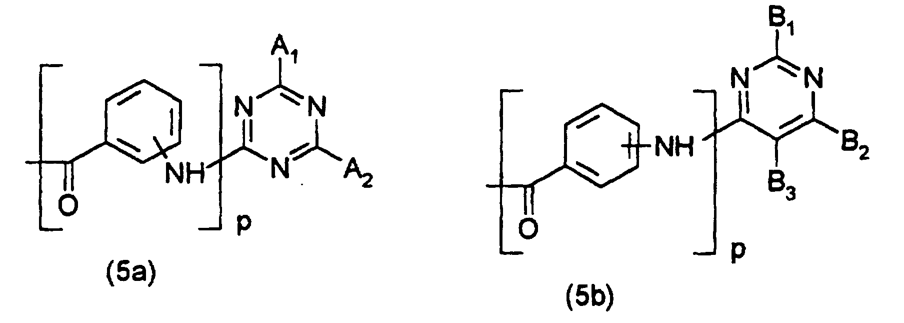 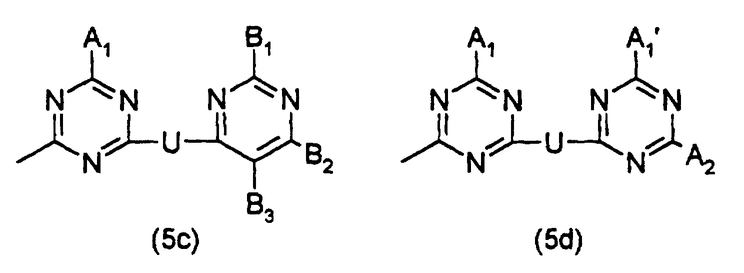 worin
- p: 0 oder 1, bevorzugt 0, ist;
- A₁: Chlor, Fluor, C₁-C₄-Alkoxy, Phenoxy, Hydroxy, Amino, Cyanamino oder gegebenenfalls durch Carboxy oder Aminocarbonyl substituiertes Pyridinyl, oder ein Rest NR₇R₈ ist, worin
R₇ Wasserstoff, C₁-C₄-Alkyl, das durch 1 bis 2, bevorzugt 1, gleiche oder verschiedene Substituenten aus der Reihe Hydroxy, Sulfo, Sulfato und Carboxy substituiert sein kann,
oder Phenyl ist, das durch 1 bis 3 gleiche oder verschiedene Substituenten aus der Reihe Methoxy, Methyl, Halogen, Sulfo oder Carboxy substituiert sein kann,
R₈ Wasserstoff oder C₁-C₄-Alkyl ist, das durch 1 bis 2, bevorzugt 1, Substituenten aus der Reihe Hydroxy, Sulfo, Sulfato und Carboxy substituiert sein kann, oder R₇ und R₈ zusammen mit dem N-Atom einen gesättigten 5- bis 7-gliedrigen Heterocyclus bilden, der noch 1 bis 2 weitere der Heterogruppen N, O, S und/oder SO₂ enthalten kann, beispielsweise Piperidin, Morpholin, Pyrrolidin, Piperazin, Thiomorpholin, Thiomorpholindioxid, insbesondere Morpholin;
- A₂: eine der Bedeutungen von A₁ hat und insbesondere Chlor, Fluor oder Cyanamino ist;
- A₁': eine der Bedeutungen von A₁ hat;
- U: ein Brückenglied aus der Reihe -NH-C₁-C₆-Alkylen-NH-, -NH-C₆-Arylen-NH-, wobei Arylen durch 1 bis 2 Reste Sulfo, Carboxy, Methyl und/oder Methoxy substituiert sein kann,
-NH-(C₆-C₁₀)-Aryl-(C₁-C₆)-alkylen-NH-, 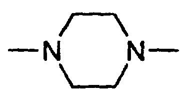 und 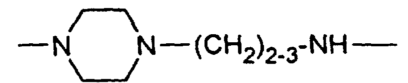 ist;
- B₁: Wasserstoff, Chlor, Fluor, Trichlormethyl, Trifluormethyl oder Methylsulfonyl ist;
- B₂: Wasserstoff, Chlor, Methyl, Methylsulfonyl oder Fluor ist;
- B₃: Wasserstoff, Cyano, Fluor oder Chlor ist,
mit der Maßgabe, daß wenigstens einer der Reste B₁ oder B₂ eine Abgangsgruppe aus der Reihe Chlor, Fluor oder Methylsulfonyl ist.

Besonders bevorzugt ist das Brückenglied U ein Rest der Formeln
-NH-CH₂-CH₂-NH-, -NH-(CH₂)₃-NH-, 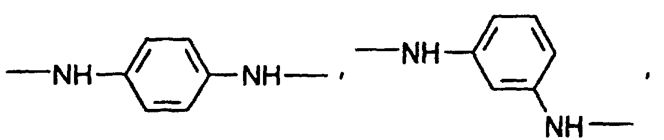 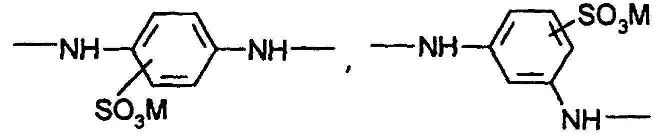 oder

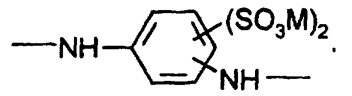

Besonders bevorzugt bedeutet der vorstehend genannte Rest NR₇R₈ eine Struktur der Formeln (6a) bis (6d) 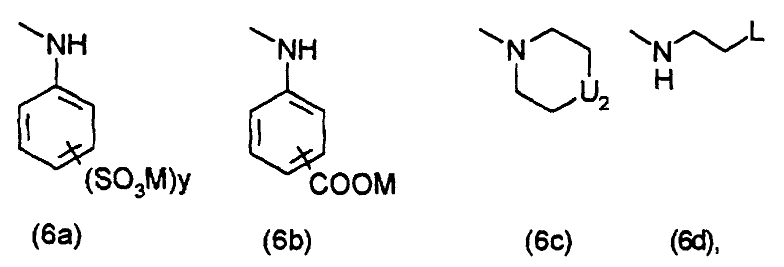 in welchen
- U₂: eine chemische Bindung, -CH₂-, -O-, -NH-, >N-(CH₂)₂OH, -S- oder -SO₂-;
- y: 1 oder 2;
- M: Wasserstoff oder ein Alkalimetall wie Li, Na oder K, und
- L: Hydroxy, Sulfo, Sulfato oder Carboxy ist.

Weiterhin bevorzugt stehen Z₁ und Z₂ für einen faserreaktiven Rest der Formel (5e) 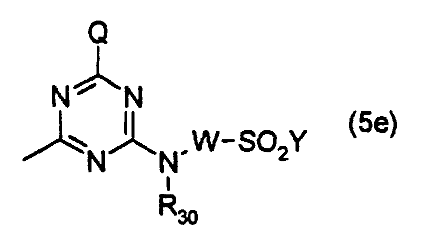 worin
- Q: Halogen, wie Chlor oder Fluor, Hydroxy, Cyanamino oder ein Rest NR₃₀-W-SO₂Y;
- W: C₂-C₆-Alkylen, das durch eine Heterogruppe O, S, NH oder SO₂ unterbrochen sein kann; Phenylen, das durch Methoxy oder Sulfo substituiert sein kann; oder Aralkylen, wie -CH₂-Phenylen- oder -Phenylen-CH₂-;
- R₃₀: Wasserstoff, C₁-C₄-Alkyl, Phenyl, das durch eine Sulfogruppe substituiert sein kann, oder der Rest -W₁-SO₂Y ist, worin W₁ C₂-C₆-Alkyl ist, und
- Y: eine der obengenannten Bedeutungen hat.

Besonders bevorzugt steht W für -(CH₂)₂-, -(CH₂)₃-, -(CH₂)₂-O-(CH₂)₂-, 1,4-Phenylen oder 1,3-Phenylen und R₃₀ für Wasserstoff, Methyl oder Phenyl.

Der Rest NR₃₀-W-SO₂Y steht besonders bevorzugt für eines der nachfolgend definierten Formeln

-NH-(CH₂)₃-SO₂Y¹ -NH-(CH₂)₂-O-(CH₂)₂-SO₂Y¹

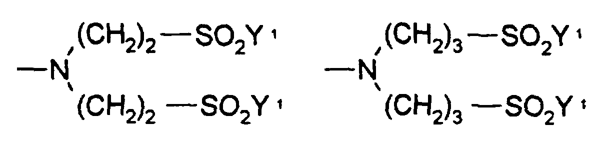

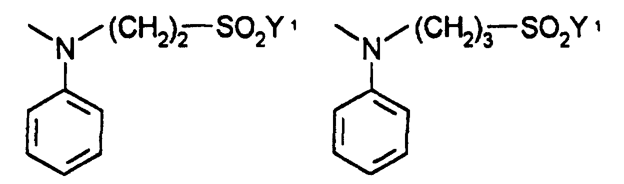

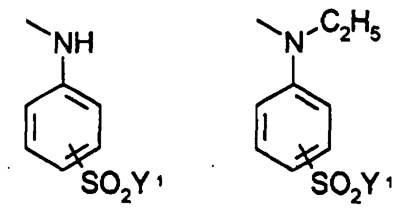

in welchen
- Y¹: Vinyl, β-Hydroxyethyl, β-Chlorethyl oder β-Sulfatoethyl ist.

Besonders bevorzugte Reste Z₁ und Z₂ sind 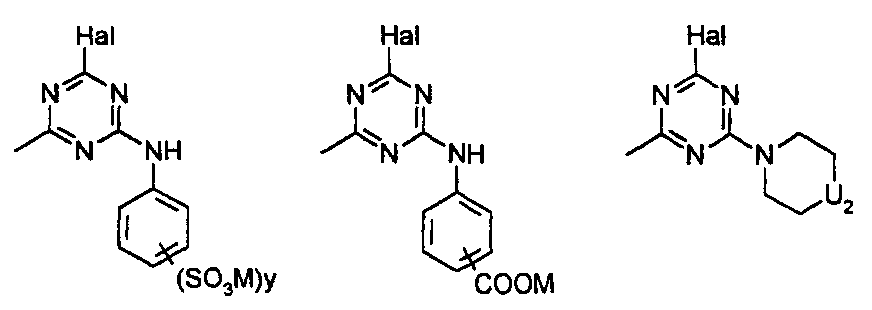 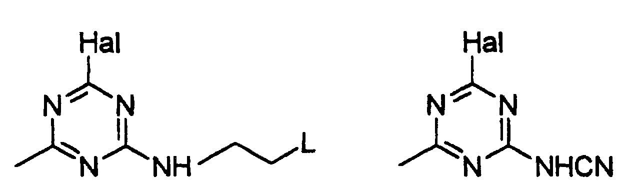 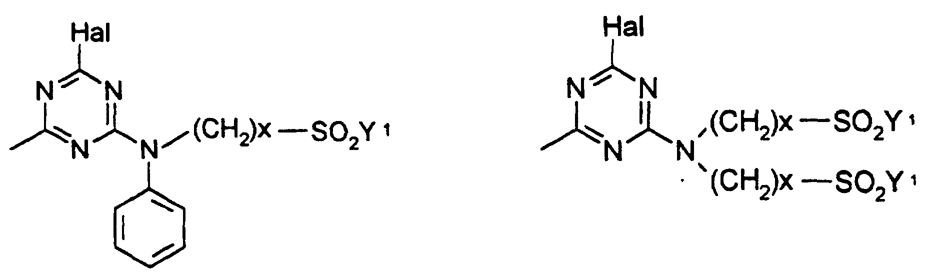 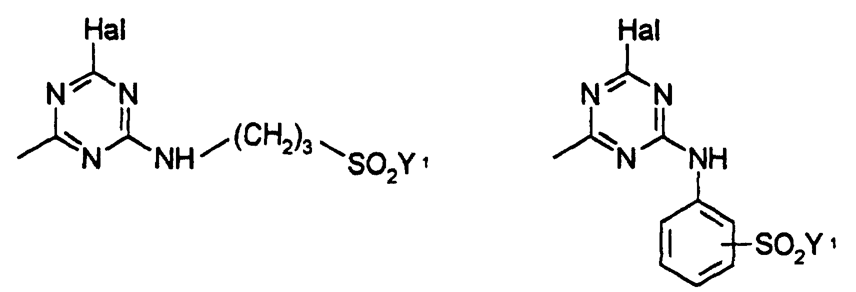 in welchen
- x: 2 oder 3 ist;
- y: 1 oder 2 ist;
- U₂: eine der obengenannten Bedeutungen hat;
- L: Hydroxy, Sulfo, Carboxy oder Sulfato ist;
- Y¹: eine der vorstehend genannten Bedeutungen hat;
- Hal: Chlor oder Fluor, und
- M: Wasserstoff oder Alkalimetall ist.

Beispiele für den Rest NR₃₀-W-SO₂Y sind
N-Phenyl-3-(β-sulfatoethylsulfonyl)-propyl-amino,
N-Methyl-2-(β-sulfatoethylsulfonyl)-ethyl-amino,
N-Phenyl-2-(β-sulfatoethylsulfonyl)-ethyl-amino,
3-(β-sulfatoethylsulfonyl)-propyl-amino,
Bis-{3-(ß-sulfatoethylsulfonyl)-propyl}-amino,
Bis-{2-(β-sulfatoethylsulfonyl)-ethyl}-amino,
Bis-{3-(ß-chlorethylsulfonyl)-propyl}-amino,
Bis-{2-(β-chlorethylsulfonyl)-ethyl)-amino,
N-Phenyl-3-(vinylsulfonyl)-propyl-amino,
N-Phenyl-2-(vinylsulfonyl)-ethyl-amino, 3-(Vinylsulfonyl)-propyl-amino,
Bis-{3-(vinylsulfonyl)-propyl}-amino, Bis-{2-(vinylsulfonyl)-ethyl}-amino,
3-(β-Sulfatoethylsulfonyl)-phenyl-amino,
4-(β-Sulfatoethylsulfonyl)-phenyl-amino,
2-Methoxy-5-(β-sulfatoethylsulfonyl)-phenyl-amino,
2,5-Dimethoxy-4-(β-sulfatoethylsulfonyl)-phenyl-amino,
2-Methoxy-5-methyl-4-(β-sulfatoethylsulfonyl)-phenyl-amino.

Beispiele für Reste der Formel Z₁ und/oder Z₂ sind
Phenylsulfonyl, 4-Methylphenylsulfonyl, 4-Acetylaminosulfonyl,
Phenylaminocarbonyl, Aminocarbonyl, Benzoyl, 2-Carboxybenzoyl,
4-Carboxybenzoyl, 3-(β-Chlorethylsulfonyl)-benzoyl, 3-Nitrobenzoyl,
4-Nitrobenzoyl, Acetyl, Propionyl, 2-Carboxy-acryloyl, 2-Carboxy-propionyl,
2,4-Dichlor-triazin-6-yl-, 2-(2'-Sulfophenyl)amino-4-chlor-triazin-6-yl-,
2-(3'-Sulfophenyl)amino-4-chlor-triazin-6-yl-,
2-(4'-Sulfophenyl)amino-4-chlor-triazin-6-yl-,
2-(2',5'-Disulfophenyl)amino-4-chlor-triazin-6-yl-,
2-Cyanamino-4-chlor-triazin-6-yl-, 2-Phenoxy-4-chlor-triazin-6-yl-,
2-Methoxy-4-chlor-triazin-6-yl-, 2-Amino-4-chlor-triazin-6-yl-,
2-(β-Sulfoethyl)amino-4-chlor-triazin-6-yl-,
2-(N-β-Sulfoethyl-N-methyl-)amino-4-chlor-triazin-6-yl-,
2-(β-Sulfoethyl)amino-4-fluor-triazin-6-yl-,
2-(N-β-Sulfoethyl-N-methyl-)amino-4-fluor-triazin-6-yl-,
Bis-{2,4-(β-Sulfoethyl)amino)-triazin-6-yl-,
Bis-{2,4-(β-Hydroxyethyl)amino}-triazin-6-yl-, 2,4-Dicyanamino-triazin-6-yl-,
2-(β-Sulfoethyl)amino-4-cyanamino-triazin-6-yl-,
4-Fluor-2-{N-phenyl-2'-(β-sulfatoethyl)sulfonyl-ethyl-amino}-triazin-6-yl-,
4-Fluor-2-{N-phenyl-3'-(β-sulfatoethyl)sulfonyl-propyl-amino}-triazin-6-yl-,
4-Fluor-2-{N-methyl-2'-(β-sulfatoethyl)sulfonyl-ethyl-amino}-triazin-6-yl-,
4-Fluor-2-{3'-(β-sulfatoethyl)sulfonyl-propyl-amino}-triazin-6-yl-,
4-Chlor-2-{N-phenyl-2'-(β-sulfatoethyl)sulfonyl-ethyl-amino}-triazin-6-yl-,
4-Chlor-2-{N-phenyl-3'-(β-sulfatoethyl)sulfonyl-propyl-amino}-triazin-6-yl-,
4-Chlor-2-{N-methyl-2'-(β-sulfatoethyl)sulfonyl-ethyl-amino}-triazin-6-yl-,
4-Chlor-2-{3'-(β-sulfatoethyl)sulfonyl-propyl-amino}-triazin-6-yl-,
4-Cyanamino-2-{N-phenyl-2'-(β-sulfatoethyl)sulfonyl-ethyl-amino}-triazin-6-yl-,
4-Cyanamino-2-{N-phenyl-3'-(β-sulfatoethyl)sulfonyl-propyl-amino}-triazin-6-yl-,
4-Chlor-2-{4'-(β-sulfatoethyl)sulfonyl-phenyl-amino}-triazin-6-yl-,
4-Cyanamino-2-{4'-(β-sulfatoethyl)sulfonyl-phenyl-amino}-triazin-6-yl-,
4-Chlor-2-{3'-(β-sulfatoethyl)sulfonyl-phenyl-amino}-triazin-6-yl-,
4-Cyanamino-2-{3'-(β-sulfatoethyl)sulfonyl-phenyl-amino}-triazin-6-yl-,
4-Chlor-2-{2'-methoxy-5'-(β-sulfatoethyl)sulfonyl-phenyl-amino}-triazin-6-yl-,
4-Cyanamino-2-{2'-methoxy-5'-(β-sulfatoethyl)sulfonyl-phenyl-amino}-triazin-6-yl-, 4-Fluor-2-{4'-(β-sulfatoethyl)sulfonyl-phenyl-amino}-triazin-6-yl-,
4-Fluor-2-{4'-(β-sulfatoethyl)sulfonyl-phenyl-amino}-triazin-6-yl-,
2,4-Difluor-pyrimidin-6-yl, 2,4-Difluor-5-chlor-pyrimidin-6-yl,
2,4,5-Trichlor-pyrimidin-6-yl, 2-Fluor-5-chlor-pyrimidin-6-yl,
5-Cyano-2,4-dichlor-pyrimidin-6-yl, 2-Methylsulfonyl-4-methyl-5-chlor-pyrimidin-6-yl, 2-Fluor-4-methyl-5-chlor-pyrimidin-6-yl, 5-Chlor-4-fluorpyrimidin-6-yl,
4,5-Difluor-2-trifluormethyl-pyrimidin-6-yl, 2,5-Dichlor-4-fluor-pyrimidin-6-yl,
2-Fluor-4,5-dichlor-pyrimidin-6-yl, 4-Fluorpyrimidin-6-yl, 2-Fluorpyrimidin-6-yl,
2-Fluor-5-chlor-pyrimidin-6-yl.

Bei dem erfindungsgemäßen Verfahren zur Herstellung der genannten beidseitig amidierten Triphendioxazinverbindungen der Formel (1) wird zunächst die Verbindung der Formel (2) zur Verbindung der Formel (3) reduziert. Die Reduktion kann mit Wasserstoff in Gegenwart eines Katalysators, wie Palladium, Platin oder Nickel, bei Temperaturen von 40 bis 90°C, oder durch Nichtedelmetalle, wie Zinn oder Zink, in Gegenwart von Säure, bevorzugt einer Mineralsäure, wie beispielsweise Salzsäure oder Schwefelsäure, bei Temperaturen von 10 bis 50°C durchgeführt werden.
Vorzugsweise wird die Reduktion mit Natriumdithionit in Wasser, insbesondere bei pH-Werten von 6 bis 9, vorzugsweise 6,5 bis 7,5, und Temperaturen von 15 bis 45°C, bevorzugt 20 bis 30°C, durchgeführt.
Das Reduktionsmittel wird zweckmäßigerweise in einer 2- bis 6-fach molaren Menge, bezogen auf die Verbindungen der Formel (2), eingesetzt.

Die Verbindungen der Formel (3) sind neu und ebenfalls Gegenstand der Erfindung.

Die Verbindungen der Formel (3) sind in saurem Medium an der Luft stabil und kann als Feststoff durch Absaugen isoliert werden. In neutralem Medium wird zweckmäßig unter Inertgas, z.B. einer Stickstoffatmosphäre, gearbeitet.

Die so erhaltenen Verbindungen der Formel (3) werden unter Inertgasatmosphäre in Wasser, in einem Lösungsmittel wie Dimethylformamid, Dimethylacetamid, N-Methyl-pyrrolidon oder in einem Wasser/Lösungsmittel-Gemisch oder in Wasser, enthaltend Hilfsmittel wie beispielsweise ε-Caprolactam, Harnstoff, Tenside, Lösemittel der obengenannten Art und andere mit Wasser mischbare Lösungsmittel wie Aceton oder Methylethylketon mit einer entsprechenden Amidierungskomponente zu Verbindungen der Formel (4) umgesetzt, wobei die vorne definierten Reste Z₁ und/oder Z₂ eingeführt werden.
Die besagten Amidierungskomponenten sind Verbindungen aus der Reihe der organischen oder anorganischen Säurehalogenide, wie Carbonsäure- oder Sulfonsäurechloride, Anhydride wie Carbonsäure- oder Sulfonsäure-Anhydride, Isocyanate, Kohlensäureester, wie Chlorkohlensäureester oder Kohlensäurediester, Harnstoffe, Carbaminsäurechloride, Carbonsäureester, Sulfonsäureester, oder elektronenarme N-haltige Heterocyclen, die Abgangsgruppen enthalten, wie insbesondere Halogentriazine oder Halogenpyrimidine.

Für den Fall, daß die Amidierungskomponente ein Carbonsäurechlorid ist, wird die Umsetzung vorteilhaft bei pH-Werten von 3 bis 9, bevorzugt 4 bis 8, und Temperaturen von 0 bis 80°C, bevorzugt 20 bis 50°C, durchgeführt.
Carbonsäurechloride sind beispielsweise Benzoylchlorid, 3- oder 4-Nitrobenzoylchlorid, Chloracetylchlorid, Acetylchlorid, Propionylchlorid oder 3-(β-Chlorethylsulfonyl)-benzoylchlorid.

Für den Fall, daß die Amidierungskomponente ein Säureanhydrid ist, wird die Umsetzung vorteilhaft bei pH-Werten von 3 bis 9, bevorzugt 4 bis 8, und Temperaturen von 30 bis 100°C, bevorzugt 40 bis 60°C, durchgeführt.

Carbonsäureanhydride sind beispielsweise Acetanhydrid oder cyclische Carbonsäureanhydride wie insbesondere Bernsteinsäureanhydrid, Maleinsäureanhydrid oder Phthalsäureanhydrid.

Für den Fall, daß die Amidierungskomponente Harnstoff oder ein Harnstoffderivat ist, wird die Umsetzung vorteilhaft in der Harnstoff(derivat)-Schmelze oder in einem hochsiedenden Lösungsmittel, wie N-Methylpyrrolidon oder Dimethylacetamid, bei Temperaturen von 120 bis 180°C durchgeführt.

Für den Fall, daß die Amidierungskomponente ein Carbaminsäurechlorid, wie CI-CO-NHC₂H₅, ist, wird die Umsetzung vorteilhaft bei pH-Werten von 4 bis 7 und Temperaturen von 40 bis 95°C in Wasser durchgeführt.

Für den Fall, daß die Amidierungskomponente ein Carbonsäureester, wie Essigsäureethylester, ist, wird die Umsetzung vorteilhaft in Wasser oder einem Wasser/Lösungsmittel-Gemisch bei Temperaturen von 90 bis 150°C und in Gegenwart von sauren oder Lewis-sauren Katalysatoren durchgeführt.

Für den Fall, daß die Amidierungskomponente ein Sulfonsäureester, wie para-Methylphenyl-SO₂-OC₂H₅, ist, wird die Umsetzung vorteilhaft in Wasser bei pH-Werten von 2 bis 5 und Temperaturen von 80 bis 150°C durchgeführt.

Für den Fall, daß die Amidierungskomponente eine 2,4-Difluor-Triazinverbindung ist, wird die Umsetzung vorteilhaft bei pH-Werten von 3 bis 9, bevorzugt 4 bis 8, und Temperaturen von 0 bis 60°C, bevorzugt 0 bis 40°C, durchgeführt.

Für den Fall, daß die Amidierungskomponente eine 2,4-Dichlor-Triazinverbindung ist, wird die Umsetzung vorteilhaft bei pH-Werten von 3 bis 10, bevorzugt 4 bis 8, und Temperaturen von 20 bis 100°C, bevorzugt 30 bis 80°C, durchgeführt.

Für den Fall, daß die Amidierungskomponente eine Monochlor-Triazinverbindung ist, wird die Umsetzung vorteilhaft bei pH-Werten von 3 bis 9, bevorzugt 4 bis 8, und Temperaturen von 50 bis 100°C, bevorzugt 60 bis 90°C, durchgeführt.

Für den Fall, daß die Amidierungskomponente eine Monofluor-Triazinverbindung ist, wird die Umsetzung vorteilhaft bei pH-Werten von 3 bis 9, bevorzugt 4 bis 8, und Temperaturen von 40 bis 90°C, bevorzugt 50 bis 70°C, durchgeführt.

Für den Fall, daß die Amidierungskomponente eine Di- oder Trifluor-Pyrimidinverbindung ist, wird die Umsetzung vorteilhaft bei pH-Werten von 3 bis 9, bevorzugt 4 bis 8, und Temperaturen von 0 bis 60°C, bevorzugt 20 bis 40°C, durchgeführt.

Für den Fall, daß die Amidierungskomponente ein Kohlensäurederivat, z.B. ein Isocyanat, ein Chlorkohlensäureester oder ein Kohlensäureester ist, wird die Umsetzung vorteilhaft bei pH-Werten von 4 bis 8, bevorzugt 5 bis 7, und Temperaturen von 20 bis 60°C, bevorzugt 25 bis 40°C, durchgeführt.

Für den Fall, daß die Amidierungskomponente ein Sulfonylchlorid ist, wird die Umsetzung vorteilhaft bei pH-Werten von 4 bis 8, bevorzugt 5 bis 7, und Temperaturen von 20 bis 60°C, bevorzugt 25 bis 40°C, durchgeführt. Sulfonylchloride sind beispielsweise 4-Methylphenyl-sulfonylchlorid, Phenylsulfonylchlorid oder 4-(Acetylamino)phenylsulfonylchlorid.

Wenn mehrere Möglichkeiten für die Herstellung gleicher Produkte beschrieben sind, so ist die Umsetzung mit Säurechloriden die bevorzugte Verfahrensweise.

Die molaren Mengenverhältnisse für die Herstellung beidseitig symmetrisch amidierter Verbindungen der Formel (4) betragen: Verbindung der Formel (3) mit T = H zur Amidierungskomponente 1:2 bis 1:6, vorzugsweise 1:2 bis 1:3.

Mischkondensationen (3) -> (4) zur Herstellung asymmetrischer Dioxazine, d.h. Z₁ ist von Z₂ verschieden, können durchgeführt werden, indem die Verbindung der Formel (2) mit T = H mit einer etwa 1:1-Mischung der den Resten Z₁ und Z₂ entsprechenden Amidierungskomponenten umgesetzt wird. Das asymmetrische Produkt wird hierbei in Mischung mit den beiden symmetrischen Kondensationsprodukten erhalten. Besser und daher bevorzugte Verfahrensweise ist jedoch die Umsetzung derjenigen Verbindung der Formel (3), worin T = Z₁ ist, mit der dem Rest Z₂ entsprechenden Amidierungskomponente. Hierbei werden wesentlich reinere einheitliche Produkte erhalten als bei der Mischkondensation, was ein besonderer Vorteil der erfindungsgemäßen Verfahrensvariante ist. Für die Umsetzung einer Verbindung der Formel (3) mit T = Z₁ mit der Amidierungskomponente Z₂ setzt man die besagte Amidierungskomponente im molaren Verhältnis von 1:1 bis 1:3, bevorzugt 1:1 bis 1:2, zur Verbindung der Formel (3) ein.

Die Verbindungen der Formel (4) sind neu und ebenfalls Gegenstand der Erfindung.

Die Verbindungen der Formel (4) werden durch Oxidation in die Verbindungen der Formel (1) überführt. Diese Oxidation kann durch Luftsauerstoff bei pH-Werten von 6 bis 10 oder durch Zusatz eines Oxidationsmittels wie Wasserstoffperoxid oder Alkaliperoxodisulfat bei pH-Werten von 4 bis 7 und 20 bis 60°C, bevorzugt 25 bis 40°C erfolgen. Die Menge an zugesetztem Oxidationsmittel beträgt zweckmäßigerweise 2 bis 6 Mol-Äquivalente, bezogen auf die Verbindung der Formel (4).

Es ist überraschend, daß das erfindungsgemäße Verfahren trotz eines zusätzlichen Reduktions- und Oxidationsschrittes wesentlich höhere Ausbeuten und bessere Produktqualitäten als nach dem Stand der Technik liefert.

Das erfindungsgemäße Verfahren zur Herstellung von Triphendioxazin-Verbindungen der Formel (1) ist für solche Triphendioxazin-Verbindungen besonders bevorzugt, die der Formel (1a) entsprechen 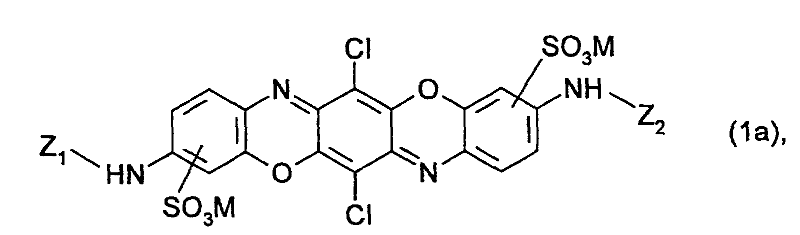 worin M für Na, K oder Li steht.

Des weiteren ist das erfindungsgemäße Verfahren zur Herstellung von solchen Triphendioxazin-Verbindungen besonders bevorzugt, die der allgemeinen Formel (7a) entsprechen 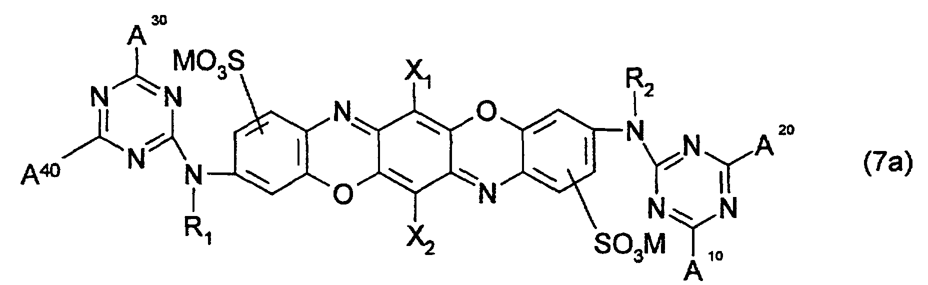 worin
- R₁, R₂, X₁, X₂ und M: eine der vorstehend genannten Bedeutungen haben,
- A¹⁰: der Rest eines C₁-C₄-aliphatischen Amins ist, das durch Sulfo, Hydroxy oder Carboxy substituiert sein kann, oder der Rest eines C₆- oder C₁₀-aromatischen Amins ist, das durch SO₂Y, 1 bis 2 Methoxygruppen und/oder 1 bis 2 Sulfogruppen substituiert sein kann, oder Cyanamino ist,
- A²⁰: Halogen ist oder eine der Bedeutungen von A¹⁰ hat,
- A³⁰: eine der Bedeutungen von A¹⁰ hat und
- A⁴⁰: eine der Bedeutungen von A¹⁰ hat oder Halogen ist.

Solche Farbstoffe sind teilweise aus EP-A-0 385 120, EP-A-0 603 823 und EP-A-0 325 246 bekannt und eignen sich beispielsweise als Reaktivfarbstoffe zum Färben von Cellulosefasern. Die in der vorliegenden Erfindung beschriebene Verfahrensweise ist vorteilhaft in Bezug auf Ausbeute und Reinheit dieser Farbstoffe.

Farbstoffe der Formel (7a), in denen beide der Reste A¹⁰ und A³⁰ für -NH-Arylen-SO₂Y und beide der Reste A²⁰ und A⁴⁰ für Fluor, Chlor, -NH-(CH₂)₂-SO₃H, -NH-Phenylen-(SO₃H)₁₋₂ oder für einen Rest -NH-Arylen-SO₂Y stehen, sind aus EP-A-0 385 120 und der japanischen Patentanmeldung Nr. 02-238063 bereits bekannt und eignen sich beispielsweise als Reaktivfarbstoffe zum Färben von Cellulosefasem. Die in der vorliegenden Erfindung beschriebene Verfahrensweise ist besonders vorteilhaft in Bezug auf Ausbeute und Reinheit dieser Farbstoffe.

Farbstoffe der Formel (7a), in denen beide der Reste A¹⁰ und A³⁰ für den Rest eines sulfierten Phenylamins wie -NH-Phenylen-(SO₃H)₁₋₂ und beide der Reste A²⁰ und A⁴⁰ für Fluor oder Chlor stehen, sind aus DE-A-2 124 080 bereits bekannt und eignen sich beispielsweise als Reaktivfarbstoffe zum Färben von Cellulosefasem. Die in der vorliegenden Erfindung beschriebene Verfahrensweise ist besonders vorteilhaft in Bezug auf Ausbeute und Reinheit dieser Farbstoffe. Diejenigen Farbstoffe der Formel (7a), in denen beide Reste A¹⁰ und A³⁰ für N(Aryl)-(CH)₂₋₃-SO₂Y und beide Reste A²⁰ und A⁴⁰ für Fluor oder Chlor stehen, sind aus EP-A-0 568 860 und DE-A-4 316 539 bereits bekannt und eignen sich beispielsweise als Reaktivfarbstoffe zum Färben von Cellulosefasern. Die in der vorliegenden Erfindung beschriebene Verfahrensweise ist besonders vorteilhaft in bezug auf Ausbeute und Reinheit dieser Farbstoffe.

Hervorzuheben ist auch von der erfindungsgemäßen Herstellung von Triphendioxazin-Farbstoffen der Formel (1) die der Formel (7a), in denen beide der Reste A¹⁰ und A³⁰ für Cyanamino und die beiden Reste A²⁰ und A⁴⁰ für Fluor, Chlor oder für einen Rest der Formeln 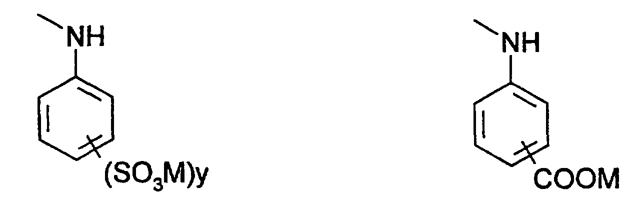 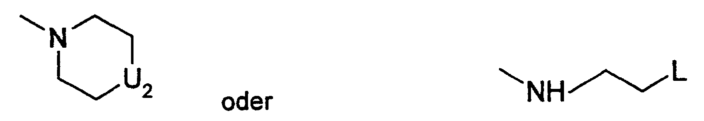 stehen, worin M, L, y und U₂ eine der vorstehend genannten Bedeutungen haben.

Des weiteren ist die erfindungsgemäße Herstellung von solchen Triphendioxazin-Farbstoffen der Formel (1) besonders bevorzugt, in denen die Reste Z₁ und Z₂ verschiedene Bedeutungen haben, beispielsweise von Verbindungen der Formel (7b) 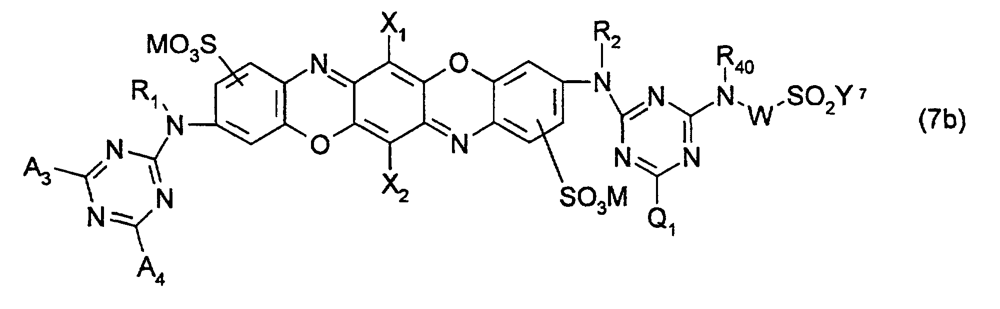 worin
- R₁, R₂, X₁, X₂, W und M: eine der vorstehend genannten Bedeutungen haben,
- R₄₀: Wasserstoff, C₁-C₄-Alkyl, Phenyl oder Sulfophenyl ist,
- Q₁: Fluor, Chlor, Amino, C₁-C₄-Alkoxy, Phenoxy oder Pyridinyl, das durch Carboxy oder Aminocarbonyl substituiert sein kann, oder Cyanamino ist,
- Y⁷: β-Sulfatoethyl, β-Chlorethyl oder Vinyl ist,
- A₃: Fluor, Chlor, Cyanamino oder Amino ist und
- A₄: einer der folgenden Reste 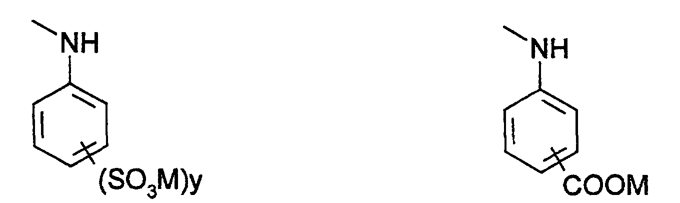 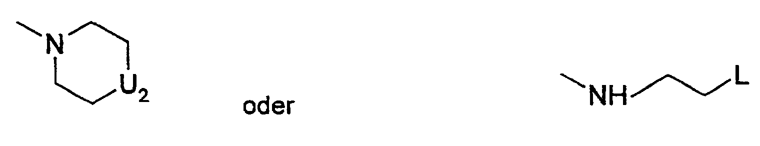 ist, worin M, U₂ ,y und L eine der vorstehend genannten Bedeutungen haben,
des weiteren von Farbstoffen der Formel (7b), in denen der Rest Q₁ Cyanamino und gleichzeitig W Phenylen ist, ebenso von Farbstoffen der Formel (7b), in denen der Rest W C₂-C₆-Alkylen ist und Q₁ eine der obengenannten Bedeutungen hat.

Diejenigen Farbstoffe der Formel (7b), in denen Q₁ für Halogen steht und W Phenylen ist, sind aus EP-A-0 385 120 bereits teilweise bekannt. Die in der Erfindung beschriebene Verfahrensweise ist vorteilhaft in Bezug auf Ausbeute und Reinheit dieser Farbstoffe.

Besonders bevorzugt ist weiterhin das erfinfungsgemäße Verfahren zur Herstellung von Farbstoffen der Formel (1), die der Formel (7c) besonders bevorzugt 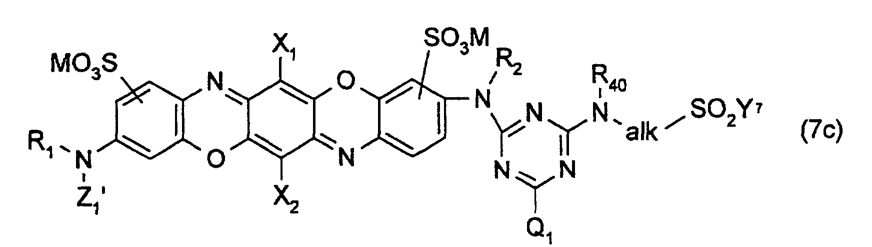 entsprechen, worin
- R₁, R₂, R₄₀, X₁, X₂, Q₁, Y⁷ und M: eine der vorstehend genannten Bedeutungen haben,
- alk: Ethylen oder Propylen und
- Z₁': C₁-C₄-Alkylcarbonyl, C₂-C₄-Alkenylcarbonyl, Aminocarbonyl, C₁-C₆-Alkylaminocarbonyl, C₆-Arylaminocarbonyl, C₆-Arylcarbonyl oder C₆-Arylsulfonyl ist, die durch Hydroxy, Cyano, Sulfo, Sulfato, Carboxy, Acetylamino oder SO₂Y⁷ substituiert sein können, hiervon Z₁' beispielsweise Acetyl, β-Carboxyacryloyl, β-Carboxypropionyl, 3-(β-Chlorethylsulfonyl)-benzoyl, Phenylsulfonyl, 4'-Methylphenylsulfonyl, Benzoyl, 2- oder 4-Carboxybenzoyl ist,
weiterhin von solchen, die der Formel (7d) 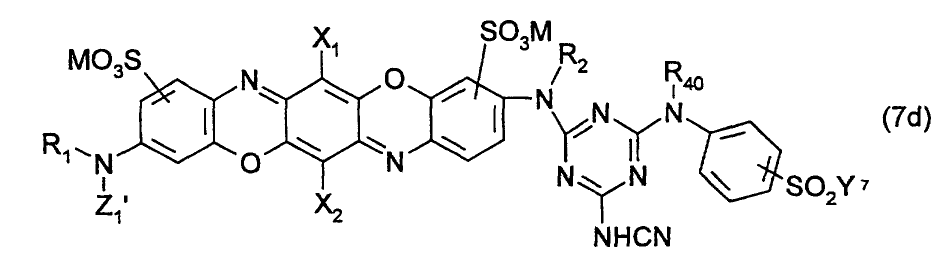 entsprechen, worin
- R₁, R₂, R₄₀, Y⁷, Z₁', X₁, X₂ und M: eine der vorstehend genannten Bedeutungen haben,
ebenso von solchen, die der Formel (7e) 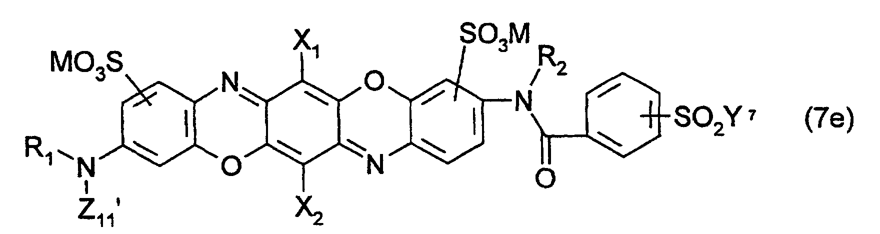 entsprechen, worin
- R₁, R₂, X₁, X₂, Y⁷ und M: eine der vorstehend genannten Bedeutungen haben,
- Z₁₁': eine der Bedeutungen von Z₁' hat oder für einen der Reste 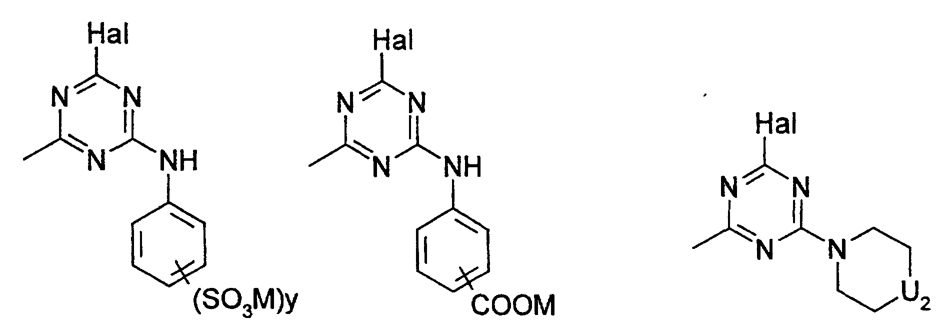 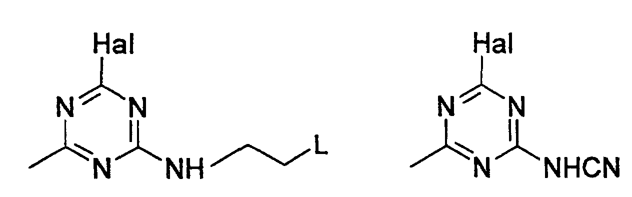
steht, worin M, y, U₂, Hal und L eine der vorstehend genannten Bedeutungen haben,
und ebenso von solchen, die der Formel (7f) 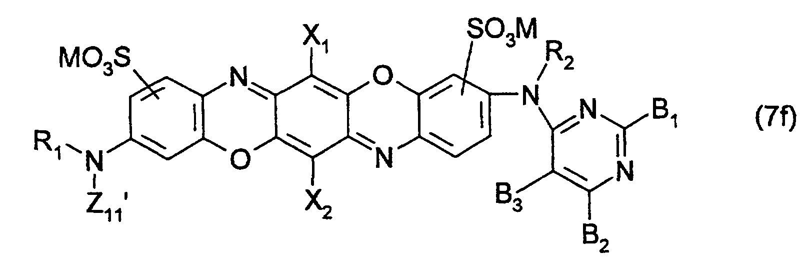 entsprechen, worin
B₁, B₂, B₃, R₁,R₂, X₁, X₂ und M eine der vorstehend genannten Bedeutungen haben und Z₁₁' eine der unter (7e) genannten Bedeutungen hat, besonders bevorzugt hiervon, in denen B₁ und B₂ jeweils Fluor ist.

Die erfindungsgemäß hergestellten Farbstoffe eignen sich zum Färben und Bedrucken von Fasermaterialien. Geeignete Fasermaterialien sind Cellulosefasern wie Baumwolle, Viskose oder chemisch modifizierte Cellulose, Polyamide wie Polyamid 6 oder Polyamid 6,6 oder Proteinfasern wie Wolle oder Seide oder Mischgewebe enthaltend mindestens eines der vorgenannten Fasermaterialien, wie Baumwolle-Polyester-Mischgewebe oder Baumwolle-Polyamid-Mischgewebe. Die Farbstoffe können als Direkt- oder Reaktivfarbstoffe auf Cellulosefasern appliziert werden, wobei Auszieh- oder Klotzfärbeverfahren wie das Klotz-Kurzverweil-Verfahren oder das Klotz-Dämpf-Verfahren, besonders bevorzugt aber Ausziehverfahren, geeignete Färbeverfahren sind.
Die Farbstoffe können als Säure- oder Reaktivfarbstoffe auf Polyamid- oder Proteinfasem appliziert werden.

### Beispiele

### Beispiel A:

0,1 mol (58,5 g) der Triphendioxazin-Verbindung der Formel 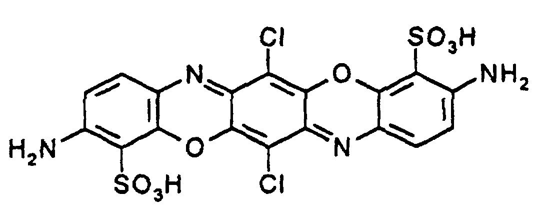 werden in 2 I fünfmolarer Salzsäure bei 25°C einige Zeit unter Stickstoffatmosphäre suspendiert. Sodann gibt man portionsweise innert 4 Stunden insgesamt 20 g Zinn hinzu.
Man rührt solange nach, bis die grünlich blaue Suspension vollständig braun geworden ist, wobei man gegebenenfalls weiteres Zinn zugeben muß, saugt dann den Ansatz ab und wäscht mit einnormaler Salzsäure nach. Der feuchte, grünlich braune Filterkuchen wird unter Stickstoff gelagert und wie in den nachfolgenden Beispielen beschrieben weiter umgesetzt.

Man erhält die Verbindung der Formel 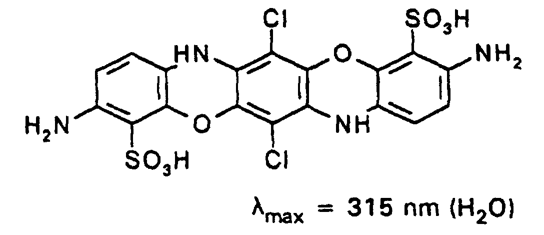 bzw. deren Hydrochlorid.

### Beispiel B:

Alternativ kann die Verbindung von Beispiel A auch hergestellt werden, indem man das Lithium-Salz der Triphendioxazin-Verbindung aus Beispiel A, hergestellt durch Lösen der Sulfosäure mit Lithiumhydroxid in 2,5 L Wasser mit 38 g Natriumdithionit bei pH-Werten von 6,5 bis 7,5 und Temperaturen von 20 bis 30°C in einer Stickstoff-Inertgasatmosphäre, umsetzt.

### Beispiel C:

Alternativ kann die Verbindung von Beispiel A auch hergestellt werden, indem man die Triphendioxazin-Verbindung bei einer Temperatur von 70°C und einem Wasserstoffdruck von 70 bar an einem Raney-Nickel-Katalysator umsetzt.

### Beispiel D:

Alternativ kann die Verbindung von Beispiel A auch hergestellt werden, indem man das Lithium-Salz der Triphendioxazin-Verbindung bei einer Temperatur von 70°C und einem Wasserstoffdruck von 70 bar an einem Palladium-Kohle-Katalysator umsetzt.

### Beispiel E:

Alternativ kann die Verbindung von Beispiel A auch hergestellt werden, indem man die Triphendioxazin-Verbindung bei einer Temperatur von 20 bis 30°C in 2 Litern zweinormaler Salzsäure und 25 g Zink umsetzt.

### Beispiel F:

67 g des Lithiumsalzes der Verbindung der Formel 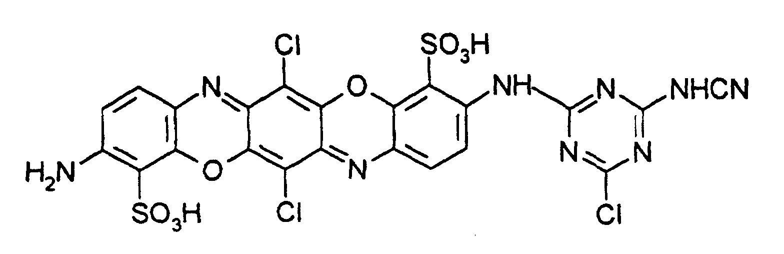 deren Herstellung in PCT/US 94/03233 beschrieben ist, werden in 2 L Wasser bei einer Temperatur von 25°C mit 24 g Natriumdithionit bei einem pH-Wert von 6,5 bis 7,5 zu der Verbindung der Formel 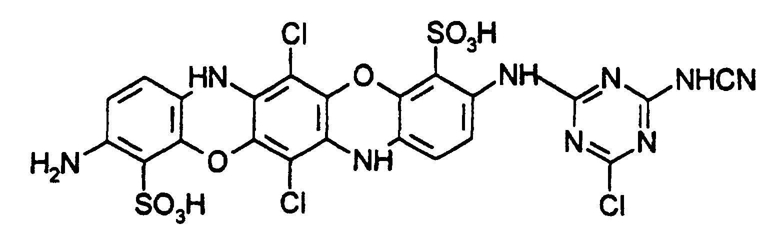 reduziert und diese Verbindung ohne Zwischenisolierung direkt weiter umgesetzt, wie in den nachfolgenden Beispielen beschrieben.

### Beispiel G:

75,4 g des Lithiumsalzes der Verbindung der Formel 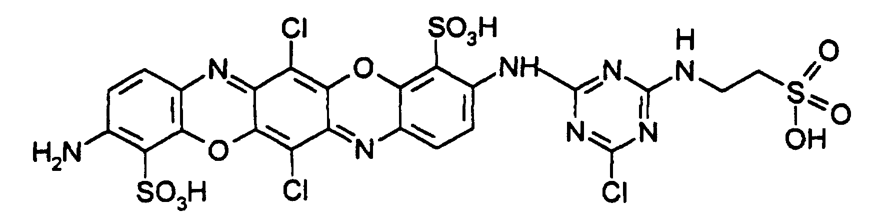 die aus der EP-A-0 385 120 / Bsp. 319 bekannt ist, wird in 1 L Wasser bei einer Temperatur von 25°C mit 38 g Natriumdithionit bei einem pH-Wert von 7 zu der Verbindung der Formel 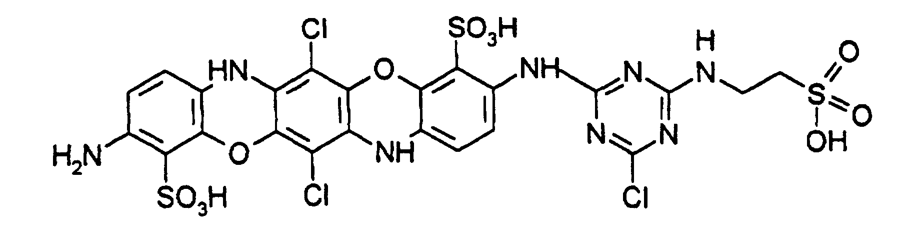 reduziert und diese Verbindung ohne Zwischenisolierung direkt weiter umgesetzt, wie in den nachfolgenden Beispielen beschrieben.

### Beispiel H:

58,9 g des Lithiumsalzes der Triphendioxazin-Verbindung
9,13-Dichlor-3,10-diamino-4,11-disulfo-triphendioxazin werden in 2 L Wasser bei einem pH-Wert von 7 und einer Temperatur von 20 bis 25°C mit 15 Teilen Acetanhydrid zu der Verbindung der Formel 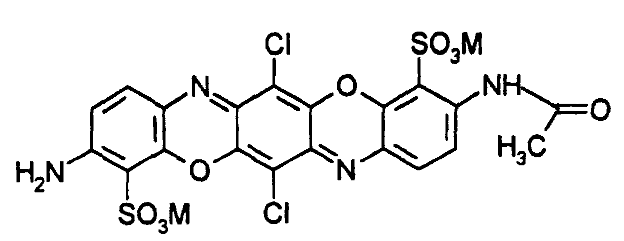 umgesetzt (M=Li).
Diese Verbindung wird mit 38 g Natriumdithionit bei einem pH-Wert von 6 bis 7 und 30°C in einer Stickstoff-Atmosphäre zu einer Verbindung der Formel 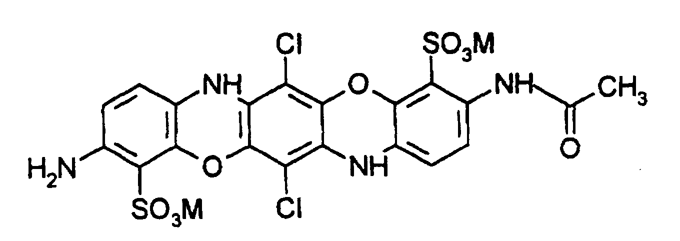 reduziert. Man rührt solange nach, bis die blaue Lösung vollständig braun geworden ist, und setzt dann die erhaltene Lösung wie in den nachfolgenden Beispielen beschrieben weiter um.

### Beispiel I:

0,1 mol (58,9 g) des Lithiumsalzes der Triphendioxazin-Verbindung
9,13-Dichlor-3,10-diamino-4,11-disulfo-triphendioxazin werden in 2 L Wasser bei einem pH-Wert von 6 bis 7 und einer Temperatur von 40 bis 50°C mit 0,25 mol (37 g) Phthalsäureanhydrid zu der Verbindung der Formel 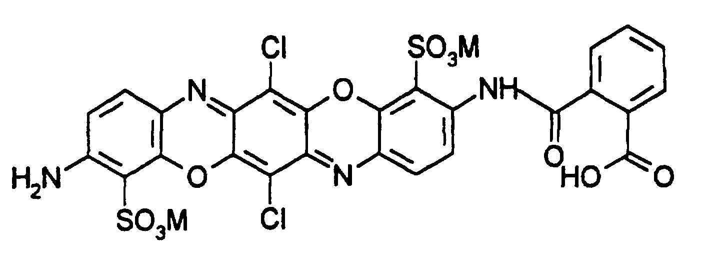 umgesetzt (M=Li).
Diese Verbindung wird mit 37 g Natriumdithionit bei einem pH-Wert von 6 bis 7 und 35°C in einer Stickstoff-Atmosphäre zu einer Verbindung der Formel 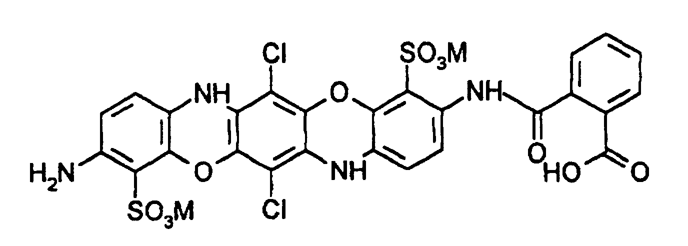 reduziert.
Man rührt solange nach, bis die blaue Lösung vollständig braun geworden ist, und setzt dann die erhaltene Lösung wie in den nachfolgenden Beispielen beschrieben weiter um.

Analog können die in der Tabelle 1 genannten Verbindungen nach einer der oben beschriebenen Verfahrensweisen hergestellt werden.

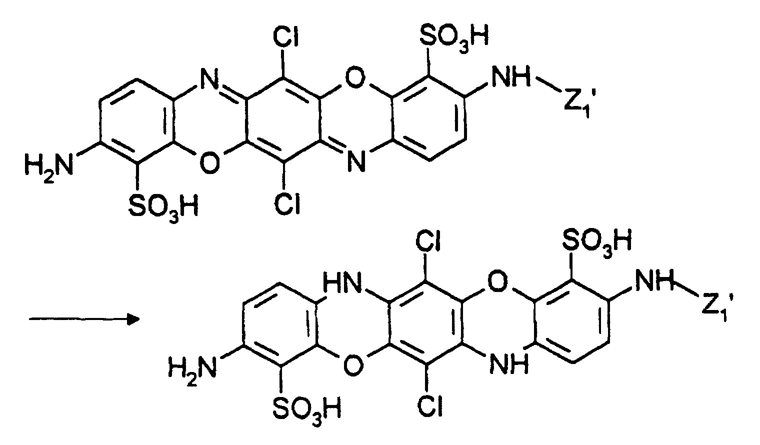

**Tabelle 1:**

| Beispiel Nr. | Z₁' |
|---|---|
| J | 5-Chlor-2,4-difluor-pyrimidyl |
| K | 2,4-difluor-pyrimidyl |
| L | Benzoyl |
| M | 3*'*-Nitrobenzoyl |
| N | 4*'*-Nitrobenzoyl |
| O | 2-Fluor-4-(4*'*-sulfophenyl)amino-triazin-6-yl |
| P | 2-Chlor-4-(3*'*-sulfophenyl)amino-triazin-6-yl |
| Q | β-Carboxypropionyl |
| R | β-Carboxyacryloyl |
| S | 4-Chlor-2-{N-Phenyl-3*'*-(β-sulfatoethylsulfonyl)-propyl-amino}triazin-6-yl |

### Beispiel T:

0,1 mol (58,5 g) der Triphendioxazin-Verbindung der Formel 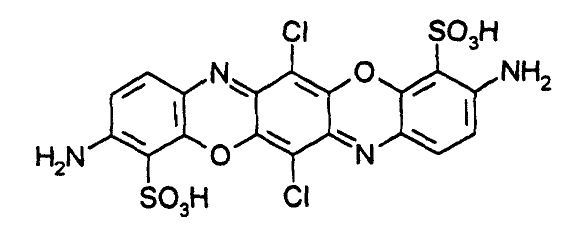 wird in 1 L 20 % Oleum einige Zeit bei 80 bis 100°C erhitzt, abkühlen gelassen und bei 0 bis 10°C auf Eis gerührt. Man saugt ab, schlämmt den Filterkuchen in Wasser an und gibt soviel Natronlauge hinzu, bis ein pH-Wert von 1 bis 2 erreicht ist. Anschließend saugt man erneut ab und isoliert den Triphendioxazinkörper der Formel 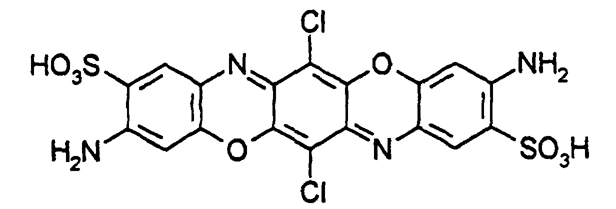 0,1 mol der auf die beschriebene Weise hergestellten Dioxazinverbindung werden in 2 L fünfmolarer Salzsäure bei 25°C einige Zeit unter Stickstoffatmosphäre suspendiert. Sodann gibt man portionsweise innert 4 Stunden insgesamt 20 g Zinn hinzu. Man rührt solange nach, bis die grünlich blaue Suspension vollständig braun geworden ist, saugt dann den Ansatz ab und wäscht mit einnormaler Salzsäure nach. Der feuchte, grünlich braune Filterkuchen wird unter Stickstoff gelagert und wie in den nachfolgenden Beispielen beschrieben weiter umgesetzt.

Man erhält die Verbindung der Formel 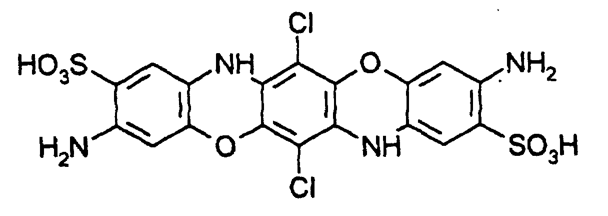 die wie in den nachfolgenden Beispielen beschrieben weiter umgesetzt wird.

### Beispiel 1:

0,1 mol der nach Beispiel A hergestellten Leuko-Dioxazin-Verbindung werden in 2 L Wasser bei einer Temperatur von etwa 60°C und einem pH-Wert von 4 bis 5 mit 0,3 mol 2-{2'-Sulfo-phenyl)-amino-4,6-dichlor-triazin versetzt und unter Beibehaltung dieses pH-Wertes und dieser Temperatur unter Stickstoffatmosphäre umgesetzt. Man entfernt sodann die Stickstoffatmosphäre und rührt noch solange an der Luft weiter, bis die Rückoxidation vollständig ist. Man erhält den Farbstoff der Formel 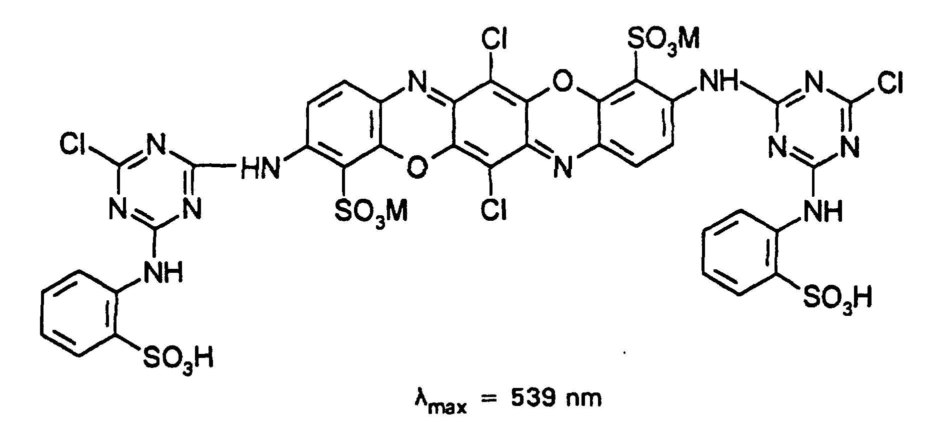 der durch Aussalzen mit Natriumchlorid isoliert wird. Der Farbstoff färbt Baumwolle in farbstarken brillanten violetten Farbtönen.

### Beispiel 2:

0,1 mol der nach Beispiel B hergestellten Leuko-Dioxazin-Verbindung werden in 2 L Wasser bei einer Temperatur von etwa 60°C und einem pH-Wert von 4 bis 5 mit 0,25 mol 2-(2',5'-Disulfo-phenyl)-amino-4,6-dichlor-triazin versetzt und unter Beibehaltung von pH und Temperatur unter Stickstoffatmosphäre umgesetzt. Man entfernt sodann die Stickstoffatmosphäre und rührt noch solange an der Luft weiter, bis die Rückoxidation vollständig ist. Man erhält den Farbstoff der Formel 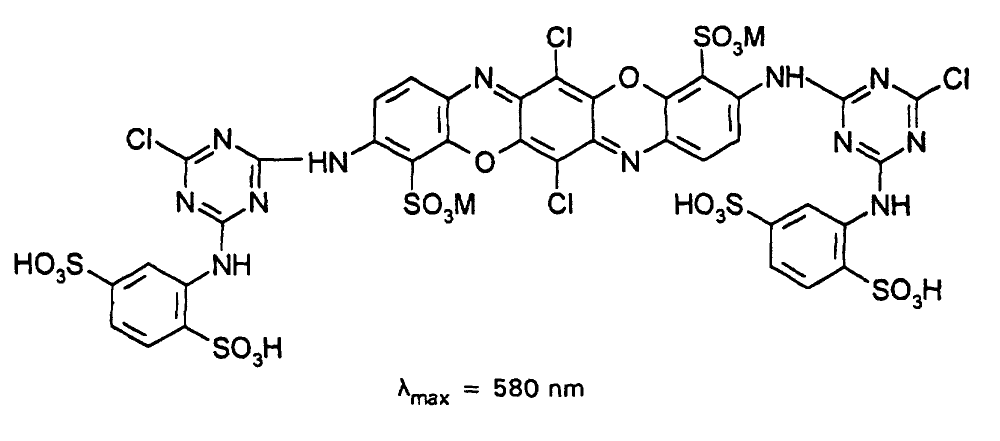 der durch Aussalzen mit Natriumchlorid isoliert wird. Der Farbstoff färbt Baumwolle in farbstarken brillanten violetten Farbtönen.

### Beispiel 3:

0,1 mol der nach Beispiel C hergestellten Leuko-Dioxazin-Verbindung werden in 2 L Wasser bei einer Temperatur von 5 bis 15°C und einem pH-Wert von 4 bis 5 mit 0,3 mol 2-(N-Phenyl-2'-{β-Sulfato-ethyl-sulfonyl}-ethyl)-amino-4,6-difluor-triazin, bekannt aus EP-A-0 568 876, versetzt und bei pH 5 bei 30 bis 40°C unter Stickstoffatmosphäre solange gerührt, bis die Umsetzung beendet ist. Man entfernt sodann die Stickstoffatmosphäre und rührt noch solange an der Luft weiter, bis die Rückoxidation vollständig ist. Man erhält den Farbstoff der Formel 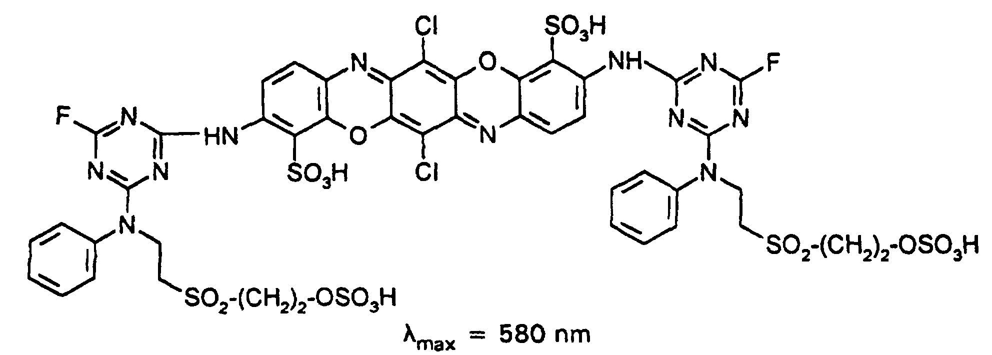 der durch Aussalzen mit Natriumchlorid isoliert wird. Der Farbstoff färbt Baumwolle in farbstarken brillanten rotstichig blauen Farbtönen.

### Beispiel 4:

0,1 mol der nach Beispiel A hergestellten Leuko-Dioxazin-Verbindung werden in 2 L Wasser bei einer Temperatur von 5 bis 15°C und einem pH-Wert von 4 bis 5 mit 0,3 mol 4,6-Dichlor-2-cyanamino-triazin versetzt und bei pH 5 bei 30 bis 40°C unter Stickstoffatmosphäre solange gerührt, bis die Umsetzung beendet ist.

Man entfernt sodann die Stickstoffatmosphäre und rührt noch solange an der Luft weiter, bis die Rückoxidation vollständig ist. Man erhält den Farbstoff der Formel 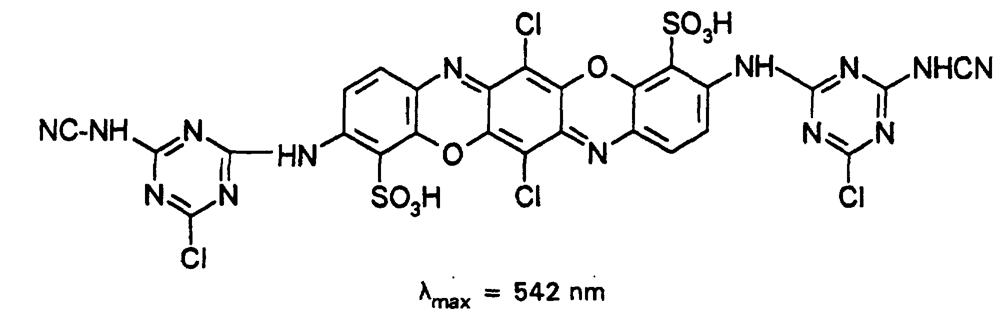 der durch Eindampfen aus der wäßrigen Syntheselösung isoliert wird. Der Farbstoff eignet sich als Reaktiv- und/oder Direktfarbstoff und färbt Cellulosefasern, wie Baumwolle, in farbstarken brillanten violetten Farbtönen.

### Beispiel 5:

0,05 mol der Verbindung aus Bsp. 4 werden in 1 L Wasser bei pH 6 und 30°C gelöst, mit 0,4 mol Morpholin versetzt und einige Zeit bei 60 bis 70°C und einem pH-Wert von 7 bis 8 gerührt, bis die Umsetzung beendet ist.
Man erhält den Farbstoff der Formel 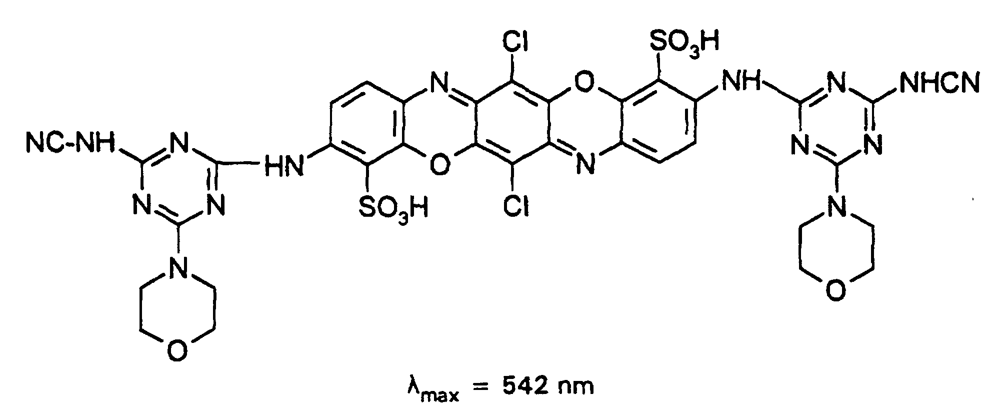 der durch Eindampfen aus der wäßrigen Syntheselösung isoliert wird. Der Farbstoff eignet sich als Direktfarbstoff und färbt Cellulosefasern, wie Baumwolle, in farbstarken brillanten violetten Farbtönen.

### Beispiele 6 bis 16

Weitere wertvolle Reaktiv- und/oder Direktfarbstoffe (siehe nachfolgende Tabelle 2) werden erhalten, wenn man analog zu den vorgenannten Beispielen verfährt.

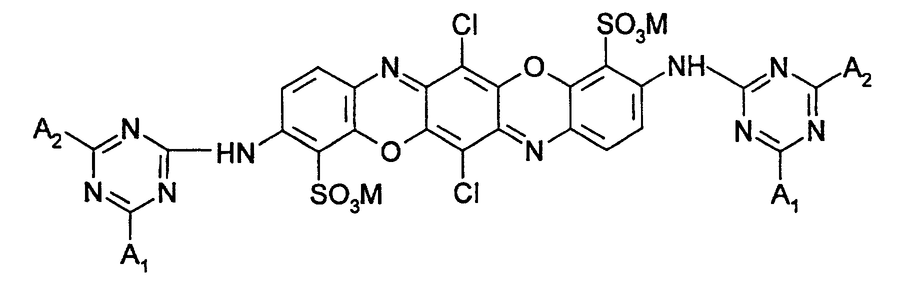

**Tabelle 2:**

| Bsp. Nr. | A₁ | A₂ | Farbton auf Baumwolle |
|---|---|---|---|
| 6 | 2-Carboxyphenyl | Chlor | violett (575 nm) |
| 6a | 3-Sulfophenyl | Chlor | violett (577 nm) |
| 6b | 4-Sulfophenyl | Chlor | violett (580 nm) |
| 7 | N-Phenyl-3'-(β-sulfatoethyl-sulfonyl)-propyl-amino | Fluor | violett (581 nm) |
| 8 | Cyanamino | Fluor | violett (578 nm) |
| 8a | Cyanamino | Cyanamino | violett (579 nm) |
| 8b | Cyanamino | N-Phenyl-3'-(β-sulfatoethyl-sulfonyl)-propyl-amino | violett (577 nm) |
| 8c | Cyanamino | 3-(β-Sulfatoethylsulfonyl)-phenyl-amino | violett (580 nm) |
| 8d | Cyanamino | N-Methyl-β-sulfoethylamino | violett (578 nm) |
| 8e | Cyanamino | β-Sulfoethylamino | violett (578 nm) |
| 9 | Amino | β-Sulfoethylamino | violett (579 nm) |
| 10 | 2-Sulfophenyl | Fluor | violett (570 nm) |
| 10a | 2-Carboxyphenyl | Fluor | violett (572 nm) |
| 10b | 3-Sulfophenyl | Fluor | violett (567 nm) |
| 10c | 3-Carboxyphenyl | Fluor | violett (570 nm) |
| 10d | 2,4-Disulfophenyl | Fluor | violett (571 nm) |
| 11 | Morpholino | Morpholino | violett (572 nm) |
| 11a | Morpholino | β-Sulfoethylamino | violett (567 nm) |
| 12 | β-Sulfoethylamino | β-Sulfoethylamino | violett (570 nm) |
| 13 | β-Sulfoethylamino | 3-Sulfophenyl | violett (571 nm) |
| 14 | Morpholino | 4-Sulfophenyl | violett (571 nm) |
| 15 | 3-(β-Sulfatoethylsulfonyl)-phenylamino | Chlor | violett (578 nm) |
| 15a | 3-(β-Sulfatoethylsulfonyl)-phenylamino | Fluor | violett (579 nm) |
| 16 | 3-(β-Sulfatoethylsulfonyl)-phenylamino | β-Sulfoethylamino | violett (579 nm) |

### Beispiel 17

0,1 mol der nach Beispiel A hergestellten Leuko-Dioxazinverbindung werden in 2 L Wasser bei einer Temperatur von etwa 40°C und einem pH-Wert von 7 mit 0,4 mol Acetanhydrid unter Stickstoffatmosphäre versetzt und einige Stunden gerührt, bis die Umsetzung vollständig ist. Man entfernt sodann die Stickstoffatmosphäre und rührt noch solange an der Luft weiter, bis die Rückoxidation vollständig ist. Man erhält den Farbstoff der Formel 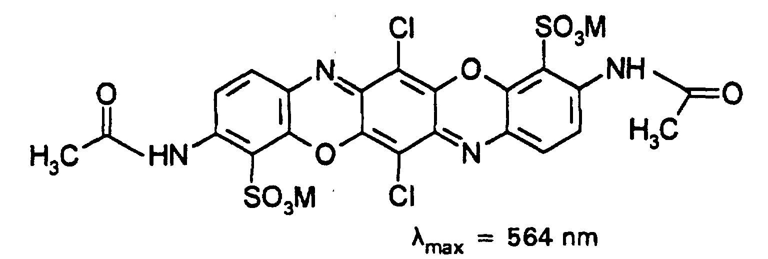 der durch Aussalzen mit Natriumchlorid isoliert wird. Der Farbstoff eignet sich als Direktfarbstoff zum Färben von Baumwolle oder Cellulose-Regeneratfasern. Weiterhin eignet sich der Farbstoff als Säurefarbstoff zum Färben von Wolle, Seide oder Polyamidfasern, wie insbesondere Polyamid-6 und Polyamid 6,6. Er färbt die genannten Fasermaterialien in farbstarken brillanten magenta-farbenen Farbtönen.

### Beispiel 18:

0,1 mol der nach Beispiel A hergestellten Leuko-Dioxazinverbindung werden in 2 L Wasser bei einer Temperatur von 40 bis 50°C und einem pH-Wert von 7 mit 0,4 mol Bernsteinsäureanhydrid unter Stickstoffatmosphäre versetzt und einige Stunden gerührt, bis die Umsetzung vollständig ist. Man entfernt sodann die Stickstoffatmosphäre und rührt noch solange an der Luft weiter, bis die Rückoxidation vollständig ist. Man erhält den Farbstoff der Formel 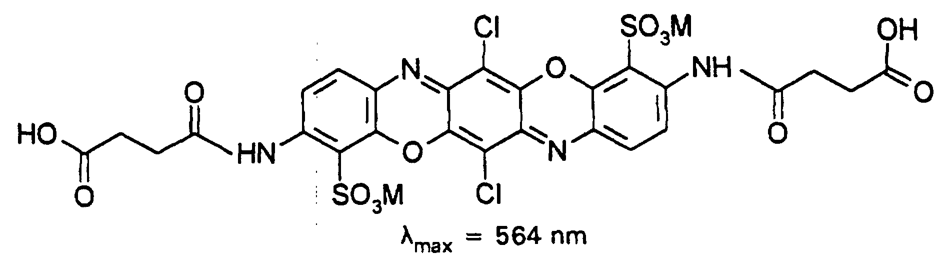 der durch Aussalzen mit Natriumchlorid isoliert wird. Der Farbstoff eignet sich als Direktfarbstoff zum Färben von Baumwolle oder Cellulose-Regeneratfasern. Weiterhin eignet sich der Farbstoff als Säurefarbstoff zum Färben von Wolle, Seide oder Polyamidfasern, wie insbesondere Polyamid-6 und Polyamid-6,6. Er färbt die genannten Fasermaterfalien in farbstarken brillanten magenta-farbenen Farbtönen. Weiterhin eignet sich der Farbstoff als Synthesezwischenprodukt für weitere Farbstoffe.

### Beispiel 19:

0,1 mol der nach Beispiel A hergestellten Leuko-Dioxazinverbindung werden in 4 L Wasser bei einer Temperatur von etwa 60°C und einem pH-Wert von 6 bis 7 mit 0,3 mol 3-Nitrobenzoylchlorid versetzt und einige Zeit unter Stickstoffatmosphäre gerührt, bis die Umsetzung beendet ist. Man entfernt sodann die Stickstoffatmosphäre und rührt noch solange an der Luft weiter, bis die Rückoxidation vollständig ist. Man erhält den Farbstoff der Formel 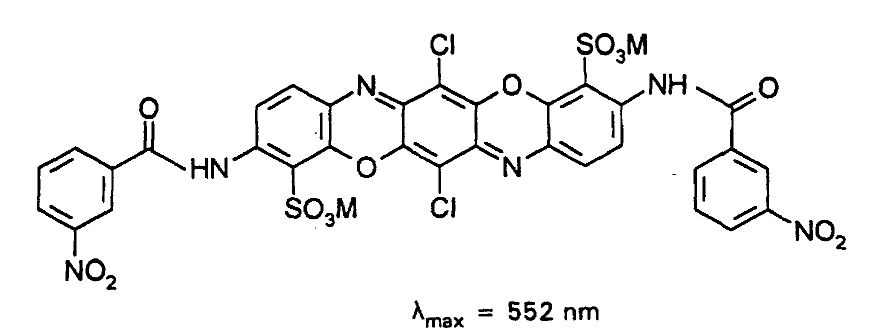 der durch Aussalzen mit Natriumchlorid isoliert wird. Der Farbstoff eignet sich als Direktfarbstoff zum Färben von Baumwolle oder Cellulose-Regeneratfasern. Weiterhin eignet sich der Farbstoff als Säurefarbstoff zum Färben von Wolle, Seide oder Polyamidfasern, wie insbesondere Polyamid-6 und Polyamid-6,6. Er färbt die genannten Fasermaterialien in farbstarken brillanten magenta-farbenen Farbtönen. Weiterhin eignet sich der Farbstoff als Synthese-Zwischenprodukt für weitere Farbstoffe.

Weitere wertvolle Direkt- und/oder Säurefarbstoffe (siehe nachfolgende Tabelle 3) werden erhalten, wenn man analog zu dem vorgenannten Beispiel verfährt.

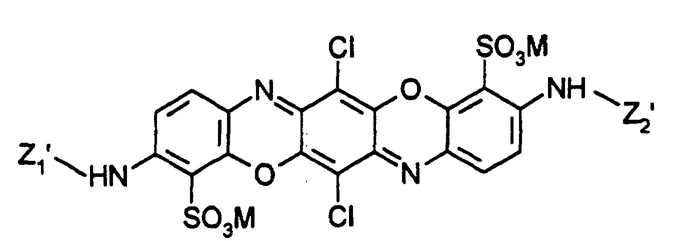

**Tabelle 3:**

| Bsp. Nr. | Z₁' | Z₂' | Farbton auf Baumwolle |
|---|---|---|---|
| 20 | Chloracetyl | Chloracetyl | magenta |
| 21 | Propionyl | Propionyl | magenta |
| 22 | 4-Nitrobenzoyl | 4-Nitrobenzoyl | magenta |
| 23 | Benzoyl | Benzoyl | magenta |
| 24 | 2-Carboxybenzoyl | 2-Carboxybenzoyl | magenta |
| 25 | 4-Carboxybenzoyl | 4-Carboxybenzoyl | magenta |
| 26 | 2-Carboxy-acryloyl | 2-Carboxy-acryloyl | magenta |

### Beispiel 27:

0,1 mol der Leukoverbindung aus Bsp. A werden in 2 L Wasser in Inertgasatmosphäre bei etwa 60°C mit 0,22 mol 4-Acetylaminophenylsulfonylchlorid bei pH 6 innert 1 h versetzt und solange weitergerührt, bis die Umsetzung vollständig ist. Man setzt sodann 0,2 mol Wasserstoffperoxid als 35%ige wäßrige Lösung hinzu, rührt an der Luft einige Zeit nach und erhält die Verbindung der Formel 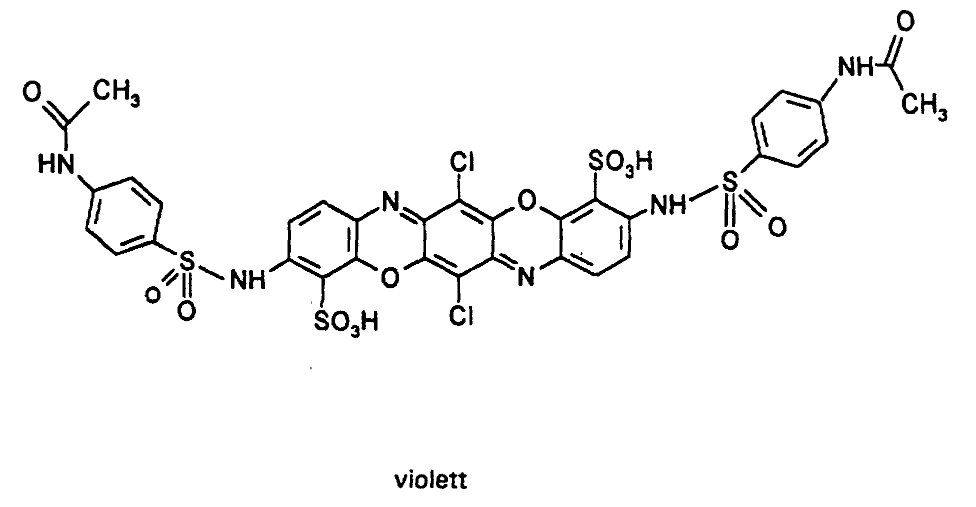 die sich als Direktfarbstoff zum Färben von Baumwolle eignet.

### Beispiel 28:

0,1 mol der isolierten und getrockneten Leukoverbindung aus Bsp. A werden in 2 L Wasser in Inertgasatmosphäre mit 7,5 mol Harnstoff in der Schmelze bei etwa 140°C so lange gerührt, bis die Umsetzung vollständig ist. Sodann gibt man die Reaktionsschmelze bei 20 bis 30°C in Wasser, setzt 0,2 mol Wasserstoffperoxid als 35%ige wäßrige Lösung hinzu und rührt an der Luft einige Zeit nach, bis die Rückoxidation vollständig ist und erhält die Verbindung der Formel 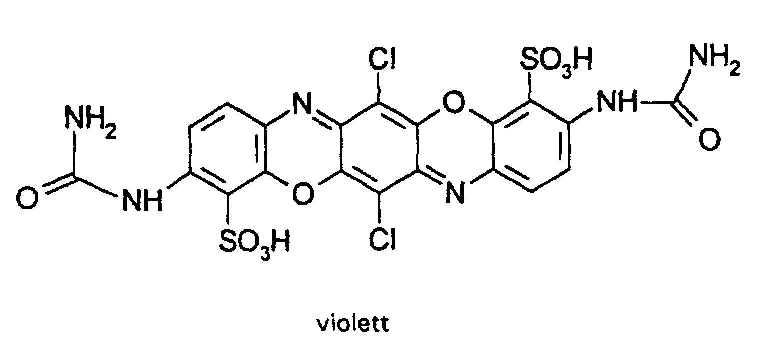 die sich als Direktfarbstoff zum Färben von Baumwolle eignet.

### Beispiel 29:

0,1 mol der nach Beispiel T hergestellten Leuko-Dioxazinverbindung werden in 2 L Wasser bei einer Temperatur von 30 bis 35°C und einem pH-Wert von 7 mit 0,4 mol Acetanhydrid unter Stickstoffatmosphäre versetzt und einige Stunden gerührt, bis die Umsetzung vollständig ist. Man entfernt sodann die Stickstoffatmosphäre und rührt noch solange an der Luft weiter, bis die Rückoxidation vollständig ist. Man erhält den Farbstoff der Formel 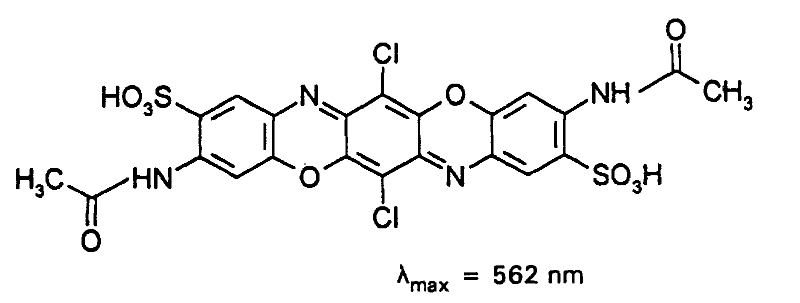 der durch Aussalzen mit Natriumchlorid isoliert wird. Der Farbstoff eignet sich als Direktfarbstoff zum Färben von Baumwolle oder Cellulose-Regeneratfasern. Weiterhin eignet sich der Farbstoff als Säurefarbstoff zum Färben von Wolle, Seide oder Polyamidfasern, wie insbesondere Polyamid-6 und Polyamid-6,6. Er färbt die genannten Fasermaterialien in farbstarken brillanten magenta-farbenen Farbtönen.

### Beispiel 30:

0,1 mol der frisch hergestellten Leuko-Verbindung aus Bsp. F werden unter Stickstoffatmosphäre in 2 L Wasser bei einem pH-Wert von 4 bis 5 bei Temperaturen von 40 bis 50°C mit 0,12 mol der Verbindung 2,4-Dichlor-6-(4'-{β-sulfatoethyl-sulfonyl}-phenyl)amino-triazin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist. Man erhält die Verbindung der Formel

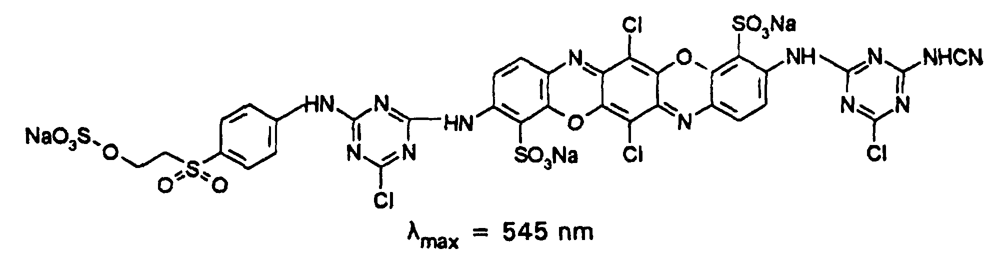

Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 31:

0,1 mol der frisch hergestellten Leuko-Verbindung aus Bsp. G werden unter Stickstoffatmosphäre in 2 L Wasser bei einem pH-Wert von 5 bis 6 und einer Temperatur von etwa 60°C mit 0,12 mol der Verbindung 2,4-Dichlor-6-(3'-β-sulfato-ethylsulfonyl-phenyl)amino-triazin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist. Man erhält die Verbindung der Formel 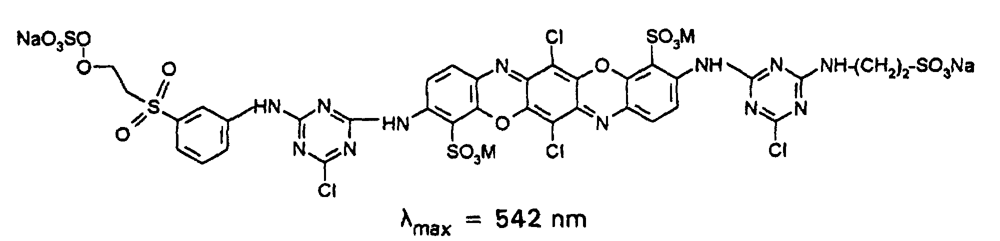 der aus der wäßrigen Syntheselösung durch Aussalzen mit Natriumchlorid isoliert wird.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 32:

0,1 mol der Verbindung aus Bsp. G wird in 2 L Wasser bei einem pH-Wert von 5 bis 6 und einer Temperatur von 50 bis 60°C mit 0,13 mol der Verbindung 2,4-Dichlor-6-(N-phenyl-2'-β-sulfato-ethylsulfonyl-ethyl)amino-triazin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist. Man erhält die Verbindung der Formel 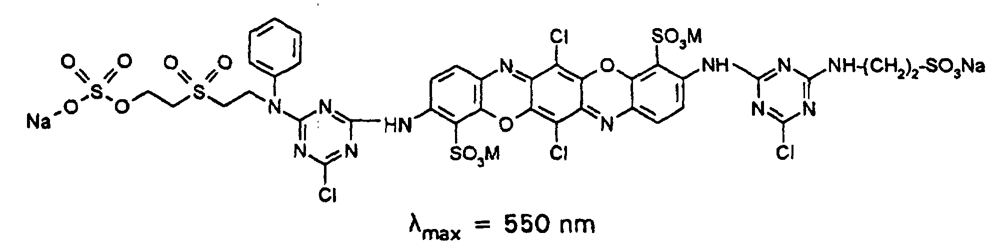 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 33:

0,1 mol der Verbindung aus Bsp. G wird in 2 L Wasser bei einem pH-Wert von 5 bis 6 und einer Temperatur von 0 bis 10°C mit 0,15 mol der Verbindung 2,4-Diffuor-6-(N-phenyl-2'-β-sulfato-ethylsulfonyl-ethyl)amino-triazin versetzt. Man rührt solange unter Erwärmung auf 25°C unter Stickstoffatmosphäre nach, bis die Umsetzung vollständig ist. Sodann wird noch einige Zeit bei 20 bis 25°C an der Luft unter Beibehaltung des pH-Werts weitergerührt, bis die Rückoxidation vollständig ist. Man erhält die Verbindung der Formel 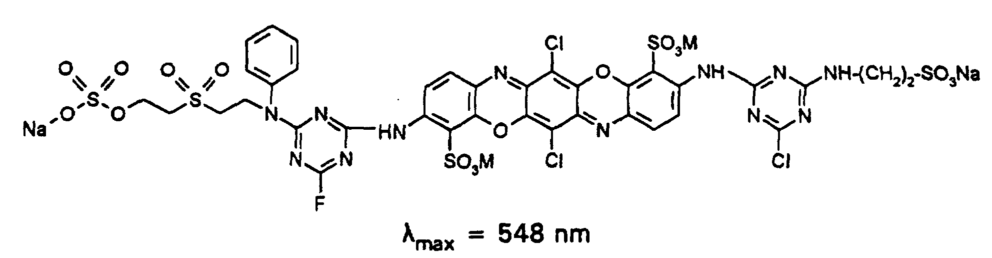 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 34:

0,1 mol der Verbindung aus Bsp.G wird in 3 L Wasser bei einem pH-Wert von 5 bis 5,5 und einer Temperatur von 60 bis 70°C mit 0,14 mol der Verbindung 2-Chlor-4-cyanamino-6-(4'-β-sulfato-ethylsulfonyl-phenyl)amino-triazin versetzt. Man rührt solange unter Stickstoffatmosphäre bei einer Temperatur von 70 bis 80°C und einem pH-Wert von 4 bis 5 nach, bis die Umsetzung vollständig ist. Sodann wird noch einige Zeit bei 20 bis 25°C an der Luft weitergerührt, bis die Rückoxidation vollständig ist. Man erhält die Verbindung der Formel 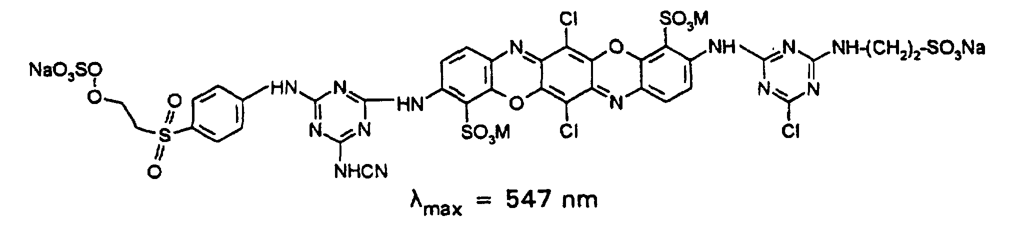 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 35:

0,1 mol des Triphendioxazin-Farbstoffs der Formel 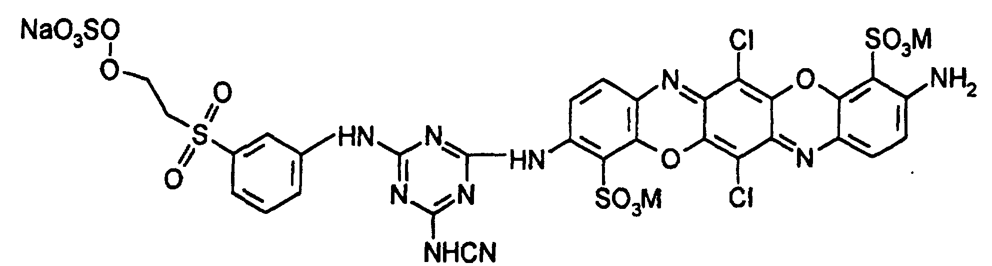 wird in 3 L Wasser bei einem pH-Wert von 5 bis 5,5 und einer Temperatur von 60 bis 70°C mit 0,14 mol der Verbindung 2,4-Dichlor-6-(2',5'-disulfophenyl)amino-triazin versetzt. Man rührt solange unter Stickstoffatmosphäre bei einer Temperatur von 70 bis 80°C und einem pH-Wert von 4 bis 5 nach, bis die Umsetzung vollständig ist. Sodann wird noch einige Zeit bei 20 bis 25°C an der Luft weitergerührt, bis die Rückoxidation vollständig ist. Man erhält die Verbindung der Formel 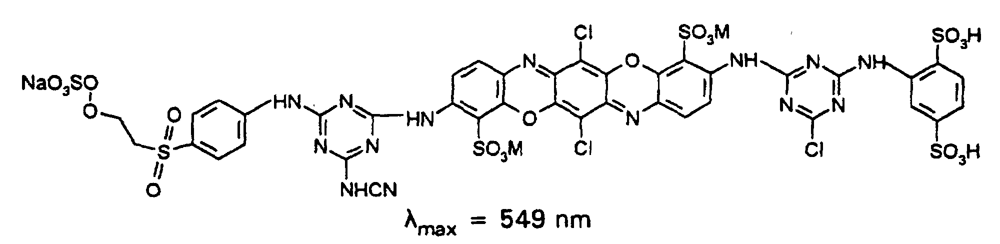 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

Weitere wertvolle Farbstoffe (siehe Tabelle 4) werden erhalten, wenn man analog zu den vorgenannten Beispielen verfährt, wobei die Reihenfolge der Umsetzungen, bezüglich der beiden Triazinreste, beliebig sein kann.

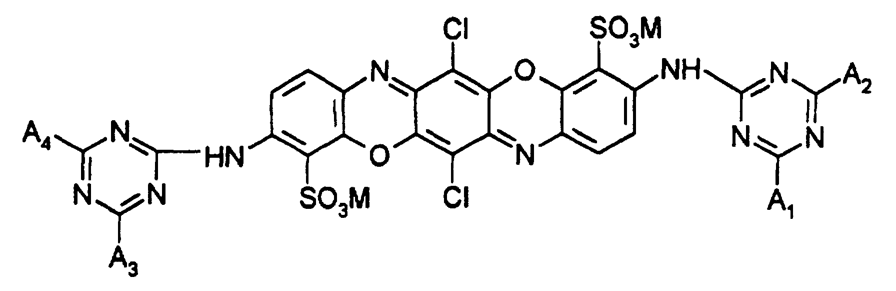

**Tabelle 4:**

| Bsp. Nr. | A₁ | A₂ | A₃ | A₄ | Farbton auf Baumwolle |
|---|---|---|---|---|---|
| 36 | Chlor | Hydroxyethyl-amino | Chlor | 3-Sulfophenyl-amino | violett |
| 37 | Chlor | N-Methyl-β-sulfoethyl-amino | Chlor | 4-Sulfophenyl-amino | violett |
| 38 | Fluor | Cyanamino | Fluor | Morpholino | violett |
| 39 | Chlor | Amino | Fluor | 2,5-Disulfo-phenyl-amino | violett |
| 40 | Chlor | N-Phenyl-2*'* -(β-sulfatoethyl-sulfonyl)-ethyl-amino | Chlor | dito | violett |
| 41 | Chlor | 3-(β-sulfatoethyl)-sulfonyl-phenyl-amino | Cyanamino | Cyanamino | violett |
| 42 | Fluor | N-Phenyl-2*'* -(β-sulfatoethyl-sulfonyl)-ethyl-amino | Chlor | Cyanamino | violett |
| 43 | Chlor | 2,5-Disulfo-phenyl-amino | Fluor | Cyanoamino | violett |
| 44 | Chlor | 2,5-Disulfo-phenyl-amino | Fluor | N-Phenyl-2*'* -(β-sulfatoethyl-sulfonyl)-ethyl-amino | violett |
| 45 | Chlor | dito | Chlor | 4-(β-sulfatoethyl)-sulfonyl-phenyl-amino | violett |

### Beispiel 46:

0,1 mol der frisch hergestellten Leuko-Verbindung aus Bsp. H wird in 2 L Wasser bei einem pH-Wert von 4 bis 5 und einer Temperatur von 20 bis 27°C mit 0,12 mol der Verbindung N-Phenyl-2,4-dichlor-6-{2'-β-sulfato-ethylsulfonylethyl}-amino-triazin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist.
Man erhält die Verbindung der Formel 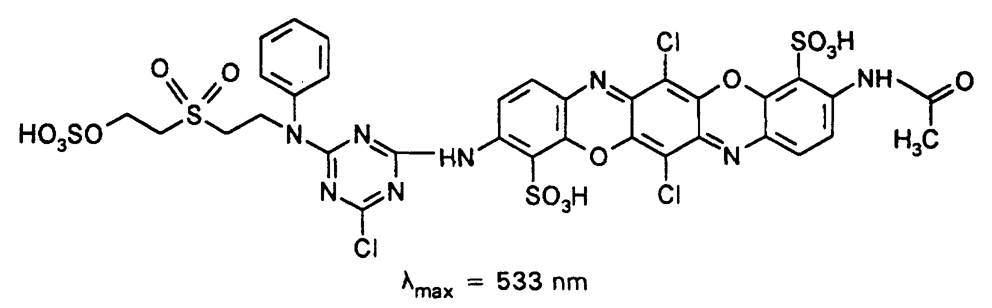 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 47:

Der Farbstoff aus dem Beispiel 46 wird ebenfalls erhalten, wenn man 0,1 mol der Verbindung aus Bsp. H in 3 L Wasser bei einem pH-Wert von 6 bis 7 und einer Temperatur von 20 bis 30°C mit 0,15 mol Cyanurchlorid, zudosiert als Lösung in Aceton, versetzt und einige Zeit bei 40°C und pH 7 nachrührt, bis die Umsetzung beendet ist. Sodann werden 0,15 mol N-Phenyl-(2-β-sulfatoethylsulfonyl)-ethyl-amin bei pH 5 hinzugesetzt und es wird einige Zeit unter Stickstoffatmosphäre bei pH 5 und etwa 50°C nachgerührt, bis die Umsetzung beendet ist. Anschließend wird der Farbstoff wie oben beschrieben rückoxidiert und wie in Beispiel 46 isoliert.

### Beispiel 48:

0,1 mol des Triphendioxazin-Farbstoffs der Formel 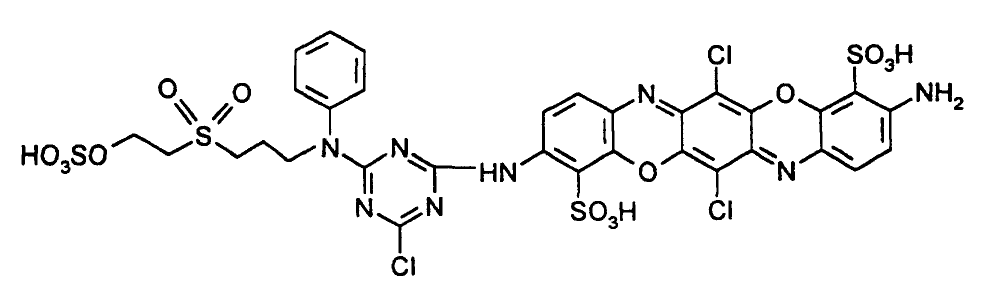 der aus EP-A1-0 628 606, Bsp. 1 bekannt ist, wird in 2 L Wasser bei einem pH-Wert von 6 bis 7 und einer Temperatur von 30 bis 40°C mit 0,15 mol Bernsteinsäureanhydrid versetzt. Man führt die Umsetzung bei 50 bis 60°C und einem pH-Wert von 7 durch und rührt solange unter Stickstoffatmosphäre nach, bis die Umsetzung vollständig ist. Sodann wird noch einige Zeit bei 20 bis 25°C an der Luft unter Zusatz von 0,1 mol Kaliumperoxodisulfat weitergerührt, bis die Rückoxidation vollständig ist. Man erhält die Verbindung der Formel 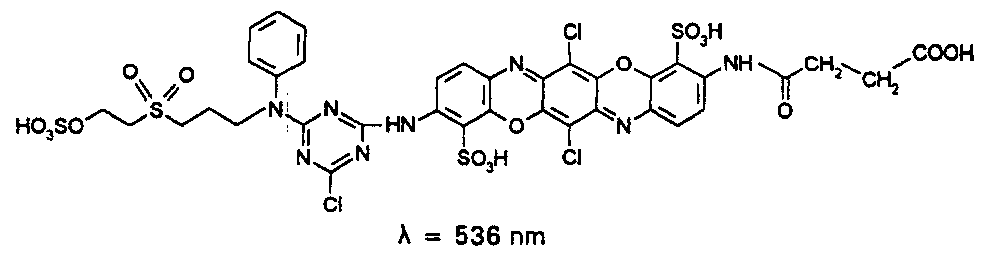 Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 49:

0,1 mol der frisch hergestellten Verbindung aus Bsp. H wird in 2 L Wasser bei einem pH-Wert von 5 bis 6 und einer Temperatur von 20 bis 25°C mit 0,14 mol der Verbindung 2,4-Difluor-6-{N-Phenyl-2'-β-sulfato-ethylsulfonyl-ethyl}-amino-triazin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist.
Man erhält die Verbindung der Formel

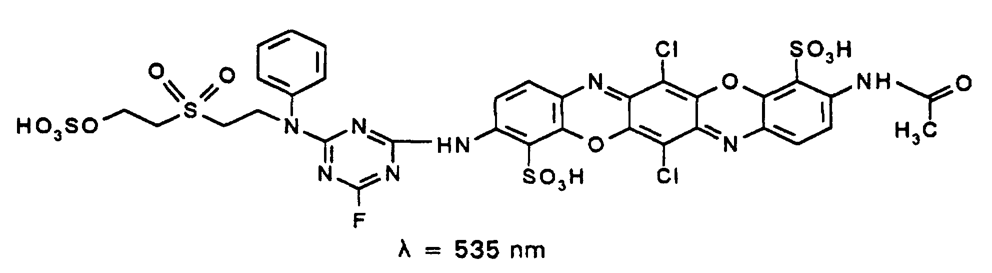

Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 20 g/l entspricht.

### Beispiel 50:

0,1 mol der Verbindung aus Bsp. F wird in 2,5 L Wasser bei einem pH-Wert von 6 und einer Temperatur von 25°C mit 0,13 mol der Verbindung 3-(β-Chlorethylsulfonyl)-benzoylchlorid versetzt. Man erwärmt sodann auf 40°C und rührt solange unter Stickstoffatmosphäre unter Beibehaltung von pH 6 bei dieser Temperatur nach, bis die Umsetzung vollständig ist. Sodann wird noch einige Zeit bei 20 bis 25°C an der Luft weitergerührt, bis die Rückoxidation vollständig ist. Man erhält die Verbindung der Formel

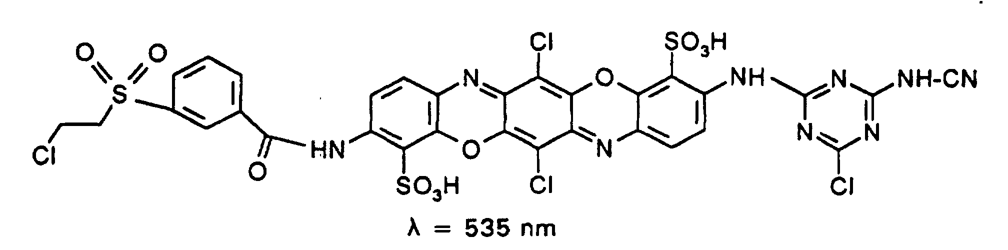

Der Farbstoff wird aus der wäßrigen Syntheselösung durch Aussalzen mit Natriumchlorid isoliert.
Der Farbstoff färbt Baumwolle in brillanten violetten Farbtönen in-guten Echtheiten. Der Farbstoff eignet sich hervorragend für Ausziehfärbungen, bei denen der Salzgehalt in der Färbeflotte 10 bis 30 g/l entspricht.

In der Tabelle 5 sind weitere Beispiele angegeben, 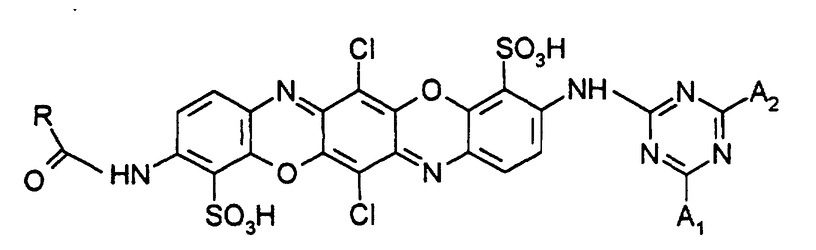 die in analoger Weise durchgeführt werden.

**Tabelle 5:**

| Bsp. Nr. | R | A₁ | A₂ | Farbton auf Baumwolle |
|---|---|---|---|---|
| 51 | β-Carboxyethyl | Chlor | N-Phenyl-2-(β-sulfatoethylsulfonyl)-ethyl-amino | violett |
| 52 | β-Carboxyvinyl | Chlor | dito | violett |
| 53 | 2-Carboxyphenyl | Chlor | dito | violett |
| 54 | Phenyl | Cyanamino | dito | violett |
| 55 | 2-Carboxyphenyl | Chlor | dito | violett |
| 56 | Methyl | Chlor | N-Phenyl-3-(β-sulfatoethylsulfonyl)-propyl-amino | violett |
| 57 | Methyl | Chlor | N-(3-Sulfo-phenyl)-3-(β-sulfatoethyl-sulfonyl)-propyl-amino | violett |
| 58 | Ethyl | Chlor | N-Phenyl-3-(β-sulfatoethylsulfonyl)-propyl-amino | violett |
| 59 | Ethyl | Fluor | dito | violett |
| 60 | Ethyl | Fluor | N-Methyl-β-Sulfoethyl-amino | violett |
| 61 | Methyl | Fluor | N-Methyl-2-(β-sulfatoethylsulfonyl)-ethylamino | violett |
| 62 | Ethyl | Fluor | dito | violett |
| 63 | β-Carboxyethyl | Fluor | dito | violett |
| 64 | β-Carboxyvinyl | Fluor | dito | violett |
| 65 | 3-(β-Chlorethyl-sulfonyl)-phenyl | Chlor | β-Sulfoethyl-amino | violett |
| 66 | dito | Fluor | β-Sulfoethyl-amino | violett |
| 67 | dito | Chlor | N-Methyl-β-Sulfoethyl-amino | violett |
| 68 | dito | Chlor | 2,5-Disulfo-phenyl-amino | violett |
| 69 | dito | Chlor | 4-Sulfo-phenyl-amino | violett |
| 70 | dito | Cyanamino | Morpholino | violett |
| 71 | dito | Cyanamino | Cyanamino | violett |
| 72 | dito | Chlor | 4-(β-sulfatoethyl)sulfonyl-phenyl-amino | violett |
| 73 | dito | Chlor | Amino | violett |
| 74 | dito | β-Sulfoethyl-amino | β-Sulfoethyl-amino | violett |
| 75 | dito | N-Methyl-β-Sulfoethyl-amino | N-Methyl-β-Sulfoethyl-amino | violett |

### Beispiel 76:

0,1 mol der frisch hergestellten Leuko-Verbindung aus Bsp. H wird in 4 L Wasser bei einem pH-Wert von 5 und einer Temperatur von 25°C mit 0,12 mol der Verbindung 3-(β-Chlorethyl-sulfonyl)-benzoylchlorid versetzt. Man erwärmt auf 40°C und rührt solange bei dieser Temperatur unter Beibehaltung des pH-Werts unter Stickstoffatmosphäre nach, bis die Umsetzung vollständig ist. Sodann wird noch einige Zeit bei 20 bis 25°C an der Luft unter Zusatz von 0,1 mol Kaliumperoxodisulfat weitergerührt, bis die Rückoxidation vollständig ist. Man erhält die Verbindung der Formel

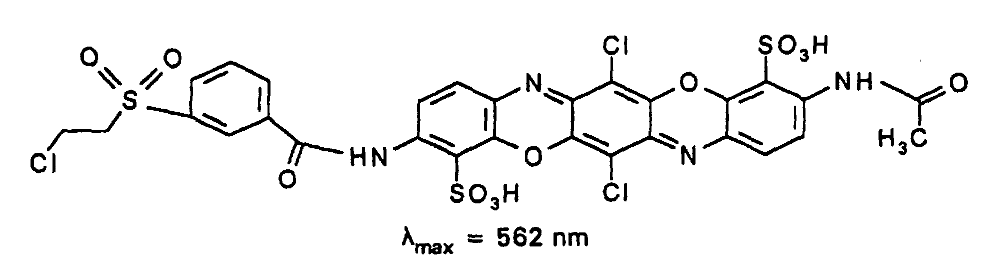

Der Farbstoff färbt Baumwolle in brillanten magenta-farbenen Farbtönen in guten Echtheiten. Der Farbstoff eignet sich hervorragend für Ausziehfärbungen, bei denen der Salzgehalt in der Färbeflotte 10 bis 20 g/l entspricht.

### Beispiel 77:

0,1 mol der Leuko-Verbindung aus Bsp. M wird in 2 L Wasser bei einem pH-Wert von 7 und einer Temperatur von 20 bis 25°C unter Stickstoffatmosphäre mit 0,12 mol Bernsteinsäureanhydrid versetzt, einige Zeit unter Beibehaltung des pH-Werts bei 35 bis 40°C nachgerührt, bis die Kondensation beendet ist. Sodann reduziert man die Nitrogruppe in der üblichen Weise mit H₂ (1 bar) an einem Pd-Kohle-Katalysator. Anschließend wird noch einige Zeit an der Luft nachgerührt, bis die Rückoxidation zur Triphendioxazin-Verbindung vollständig ist. Man erhält die Verbindung der Formel 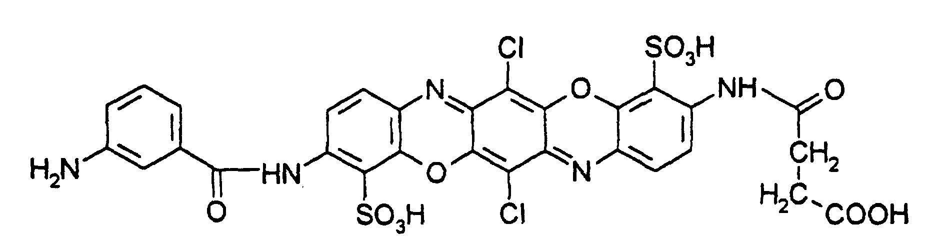 die durch Ausalzen mit Natriumchlorid isoliert wird.
Das erhaltene Produkt wird anschließend bei pH 5 bis 6 und einer Temperatur von 15 bis 20°C gelöst und sodann mit 0,1 mol 5-Chlor-2,4,6-difluor-pyrimidin versetzt. Sodann wird noch einige Zeit bei 20 bis 25°C an der Luft weitergerührt, bis die Umsetzung vollständig ist. Man erhält die Verbindung der Formel

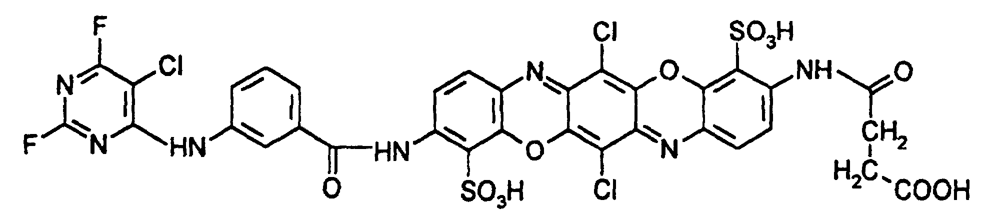

Der Farbstoff färbt Baumwolle in brillanten magenta-farbenen Farbtönen in guten Echtheiten. Der Farbstoff eignet sich hervorragend für Ausziehfärbungen, bei denen der Salzgehalt in der Färbeflotte 10 bis 20 g/l entspricht.

Die nachfolgende Tabelle 6 enthält weitere Beispiele der Formel 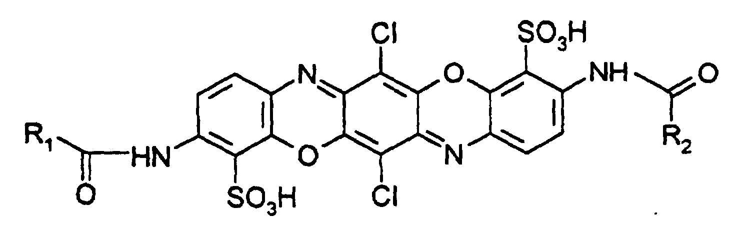 die in analoger Weise hergestellt werden.

**Tabelle 6:**

| Bsp. Nr. | R₁ | R₂ | Farbton auf Baumwolle |
|---|---|---|---|
| 78 | Ethyl | 3-(β-Chlorethylsulfonyl)-phenyl | magenta |
| 79 | Phenyl | dito | magenta |
| 80 | 4-Carboxyphenyl | dito | magenta |
| 81 | 2-Carboxyphenyl | dito | magenta |
| 82 | 2-Carboxyethyl | dito | magenta |
| 83 | 2-Carboxyvinyl | dito | magenta |
| 84 | 3-(β-Chlorethylsulfonyl)-phenyl | dito | magenta |
| 85 | Methyl | 3-(2*'* ,4*'* -Difluorpyrimidin-6*'* -yl)-amino-phenyl | magenta |
| 86 | Ethyl | dito | magenta |
| 87 | Phenyl | 3-(5*'* -Chlor-2*'* ,4*'* -difluorpyrimidin-6*'* -yl)-amino-phenyl | magenta |
| 88 | 4-Carboxyphenyl | 3-(5*'* -Cyano-2*'* ,4*'* -dichlorpyrimidin-6*'* -yl)-amino-phenyl | magenta |
| 89 | 2-Carboxyphenyl | 3-(2*'* ,4*'* ,5*'* -Trichlor-pyrimidin-6*'* -yl)-amino-phenyl | magenta |
| 90 | 2-Carboxyethyl | 4-(2*'* ,4*'* -Difluorpyrimidin-6*'* -yl)-amino-phenyl | magenta |
| 91 | 2-Carboxyvinyl | dito | magenta |
| 92 | Methyl | 3-(2*'* -β-Sulfoethyl-4*'* -fluor-triazin-6*'* -yl)-amino-phenyl | magenta |
| 93 | Ethyl | 3-(2*'* -amino-4*'* -chlor-triazin-6*'* -yl)-amino-phenyl | magenta |
| 94 | β-Carboxyethyl | 3-(2*'* -{2*''* ,5*''* -disulfophenyl}-amino-4*'* -chlor-triazin-6*'* -yl)-amino-phenyl | magenta |
| 95 | 2-Carboxy-phenyl | 3-(2*'* -{4*''* -sulfophenyl}amino-4*'* -chlor-triazin-6*'* -yl)-amino-phenyl | magenta |
| 96 | Methyl | 3-(2*'* -N-Phenyl-{2*''* -(β-sulfatoethyl)-sulfonyl-ethyl}amino-4*'* -chlor-triazin-6*'* -yl)-amino-phenyl | magenta |
| 97 | Methyl | 3-(2*'* -N-Phenyl-{3*''* -(β-sulfatoethyl)-sulfonyl-propyl}amino-4*'* -chlor-triazin-6*'* -yl)-amino-phenyl | magenta |

### Beispiel 98 :

0,1 mol der Verbindung aus Bsp. Q wird in 2 L Wasser bei einem pH-Wert von 5 bis 6 und einer Temperatur von 10 bis 20°C mit 0,14 mol 5-Chlor-2,4,6-trifluor- pyrimidin versetzt. Man erwärmt sodann auf 40°C und rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist. Man erhält die Verbindung der Formel

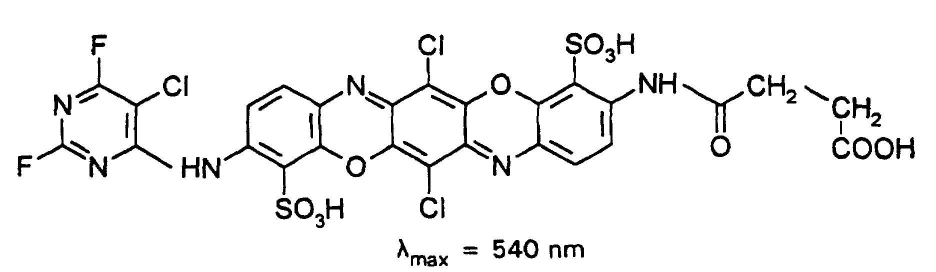

Der Farbstoff wird aus der wäßrigen Syntheselösung durch Aussalzen mit Natriumchlorid isoliert.
Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

### Beispiel 99 :

0,1 mol der Verbindung aus Bsp. F wird in 3 bis 4 L Wasser bei einem pH-Wert von 5 bis 6 und einer Temperatur von 18 bis 22°C mit 0,2 mol 5-Chlor-2,4,6-trifluor-pyrimidin versetzt. Man rührt solange unter Stickstoffatmosphäre nach, bis die Umsetzung vollständig ist. Sodann wird noch einige Zeit bei 20 bis 25°C an der Luft weitergerührt, bis die Rückoxidation vollständig ist.
Man erhält die Verbindung der Formel

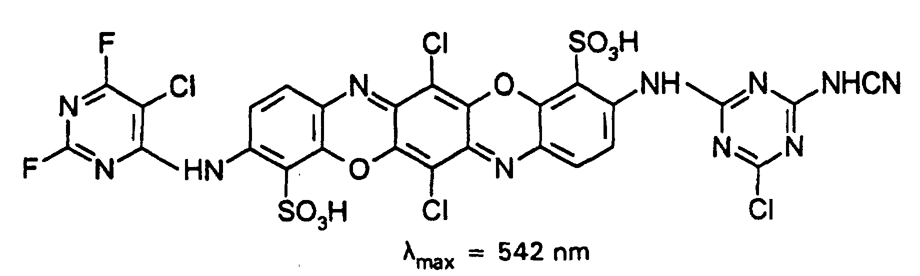

Der Farbstoff wird aus der wäßrigen Syntheselösung durch Aussalzen mit Natriumchlorid isoliert. Der Farbstoff färbt Baumwolle in brillanten violetten Farbtönen in guten Echtheiten. Der Farbstoff eignet sich hervorragend für Ausziehfärbungen, bei denen der Salzgehalt in der Färbeflotte 10 bis 30 g/l entspricht.

Die Tabelle 7 zeigt weitere wertvolle Farbstoffe der allgemeinen Formel 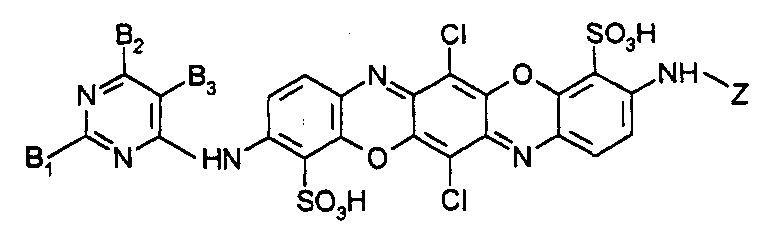 die in analoger Weise hergestellt werden.

**Tabelle 7:**

| Bsp. Nr. | B ₁ | B ₂ | B ₃ | Z | Farbton auf Baumwolle |
|---|---|---|---|---|---|
| 100 | Fluor | Fluor | Wasserstoff | Acetyl | magenta |
| 101 | Chlor | Chlor | Cyano | Acetyl | magenta |
| 102 | Chlor | Chlor | Chlor | Propionyl | magenta |
| 103 | Fluor | Wasserstoff | Chlor | Propionyl | magenta |
| 104 | Fluor | Fluor | Wasserstoff | Propionyl | magenta |
| 105 | Fluor | Fluor | Wasserstoff | β-Carboxypropionyl | magenta |
| 106 | Fluor | Fluor | Wasserstoff | dito | magenta |
| 107 | Fluor | Fluor | Wasserstoff | β-Carboxyacryloyl | magenta |
| 108 | Fluor | Fluor | Wasserstoff | dito | magenta |
| 109 | Fluor | Fluor | Chlor | 2-Chlor-4-amino-triazin-6-yl | magenta |
| 110 | Fluor | Fluor | Chlor | 2-Chlor-4-β-sulfoethyl-amino-triazin-6-yl | magenta |
| 111 | Fluor | Fluor | Wasserstoff | 2-Fluor-4-{N-methyl-β-sulfo-ethylamino}-triazin-6-yl | magenta |
| 112 | Fluor | Fluor | Wasserstoff | 2-Chlor-4-cyanamino-triazin-6-yl | magenta |
| 113 | Fluor | Wasserstoff | Chlor | 5-Chlor-2-fluor-pyrimidin-6-yl | magenta |

### Beispiel 114:

0,2 mol des Lithiumsalzes der Leuko-Verbindung aus Bsp. A wird in 4 L Wasser bei einem pH-Wert von 6,5 bis 7 bei 25°C mit 0,65 mol 5-Chlor-2,4,6-trifluor-pyrimidin versetzt, wobei zur Steuerung des pH-Werts auf pH 7 ständig Lithiumhydroxidlösung zudosiert wird. Man rührt solange nach, bis die Umsetzung vollständig ist, und isoliert den Farbstoff der Formel 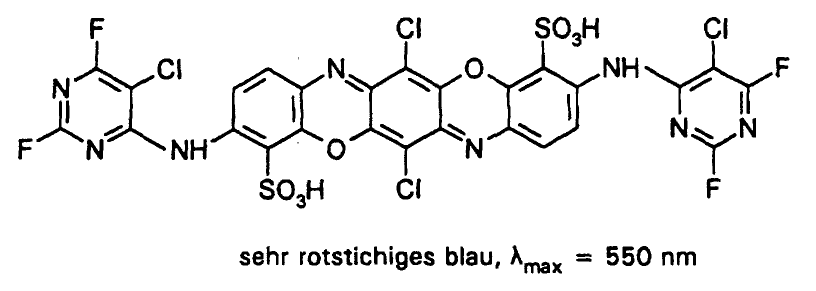 durch Aussalzen mit Natriumchlorid. Der Farbstoff färbt Baumwolle in brillanten, sehr rotstichig blauen Farbtönen in sehr hohen Farbstärken und guten Echtheiten, von denen die Waschechtheiten besonders hervorzuheben sind. Besonders vorteilhaft, insbesondere im Hinblick auf die Ökologie, ist die Tatsache, daß der Farbstoff mit sehr wenig Salzeinsatz, z.B. mit 5 bis 10 g/l Natriumsulfat, gefärbt werden kann.

### Beispiel 115:

0,2 mol des Lithiumsalzes der Leuko-Verbindung aus Bsp. A wird in 3,5 L Wasser bei einem pH-Wert von 6,8 bis 7,2 bei etwa 27°C mit 0,5 mol 2,4,6-Trifluor-pyrimidin versetzt, wobei zur Steuerung des pH-Werts auf pH 7 ständig Lithiumhydroxidlösung zudosiert wird. Man rührt solange nach, bis die Umsetzung vollständig ist, und isoliert den Farbstoff der Formel 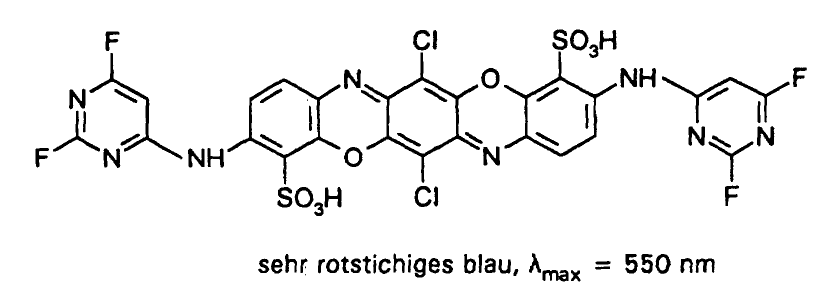 durch Aussalzen mit Natriumchlorid. Der Farbstoff färbt Baumwolle in brillanten, sehr rotstichig blauen Farbtönen in sehr hohen Farbstärken und guten Echtheiten, von denen die Waschechtheiten besonders hervorzuheben sind.

Besonders vorteilhaft, insbesondere im Hinblick auf die Ökologie, ist die Tatsache, daß der Farbstoff mit sehr wenig Salzeinsatz, z.B. mit 5 bis 10 g/l Natriumsulfat, gefärbt werden kann.

Weitere Reaktivfarbstoffe werden erhalten (siehe nachfolgende Tabelle 8), wenn man analog der vorhergehenden Beispiele verfährt.

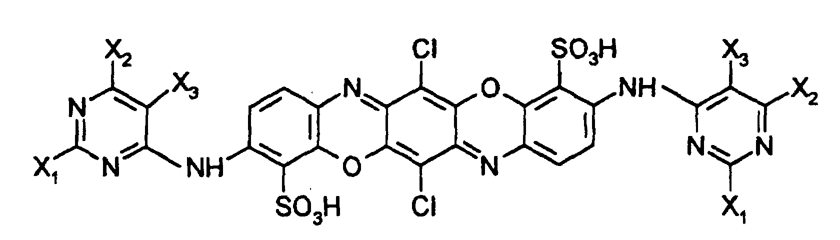

**Tabelle 8:**

| Bsp.Nr. | X₁ | X₂ | X₃ | Farbton |
|---|---|---|---|---|
| 116 | Chlor | Chlor | Chlor | sehr rotstichiges blau |
| 117 | Chlor | Chlor | Cyano | dito |
| 118 | Fluor | Fluor | Cyano | dito |
| 119 | Methylsulfonyl | Methyl | Chlor | dito |
| 120 | Wasserstoff | Fluor | Chlor | dito |

## Patentansprüche

1. Verfahren zur Herstellung von Triphendioxazin-Verbindungen der allgemeinen Formel (1) 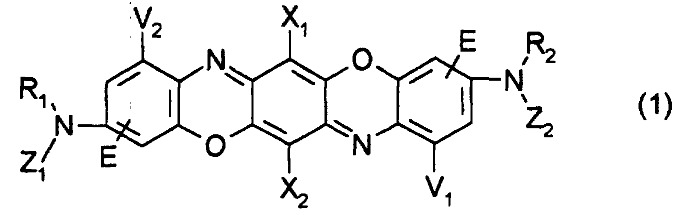 worin
R₁ Wasserstoff oder C₁-C₄-Alkyl ist, das durch 1 bis 2 Substituenten aus der Reihe Hydroxy, C₁-C₄-Alkoxy, Sulfato oder Sulfo substituiert sein kann;
R₂ eine der Bedeutungen von R₁ hat;
E Sulfo, Carboxy, C₁-C₄-Alkylsulfonyl oder ein Rest SO₂Y ist, in welchem Y Vinyl oder CH₂CH₂V ist, worin V für Hydroxy oder für eine Abgangsgruppe aus der Reihe Sulfato, Phosphato, Thiosulfato oder Halogen steht;
oder -SO₂NR₃R₄ oder -CONR₃R₄ ist, worin
R₃ Wasserstoff, Phenyl, oder C₁-C₄-Alkyl ist, das durch Hydroxy, Carboxy, Sulfo, Sulfato oder einen Rest SO₂Y substituiert sein kann,
R₄ eine der Bedeutungen von R₃ hat, oder zusammen mit R₃ und N einen 5- oder 6-gliedigen Heterocyclus bildet, der durch 1 bis 3 weitere Heteroatome aus der Reihe N, O und S unterbrochen sein kann;
X₁ Halogen, Wasserstoff, C₁-C₆-Alkyl, Phenyl, Phenoxy oder C₁-C₄-Alkoxy ist;
X₂ eine der Bedeutungen von X₁ hat;
V₁ Wasserstoff, Sulfo, Methoxy, Methyl oder Halogen ist;
V₂ eine der Bedeutungen von V₁ hat; und
Z₁ und Z₂ gleich oder verschieden sind und einen Acylrest, einen unsubstituierten, alkylierten oder arylierten Aminocarbonylrest, einen Sulfonylrest oder einen stickstoffhaltigen heteroaromatischen Rest bedeuten,
**dadurch gekennzeichnet, daß** man eine Verbindung der Formel (2) 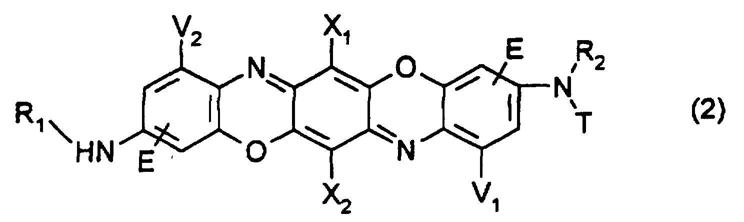 worin T Wasserstoff, Z₁ oder Z₂ ist,
zu einer Verbindung der Formel (3) reduziert, 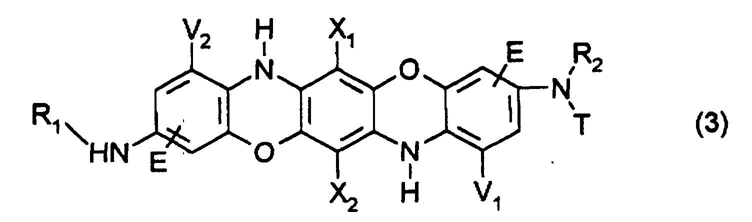 diese mit einem dem Rest Z₁ und/oder Z₂ zugrundeliegenden reaktiven Derivat zu einer Verbindung der Formel (4) umsetzt 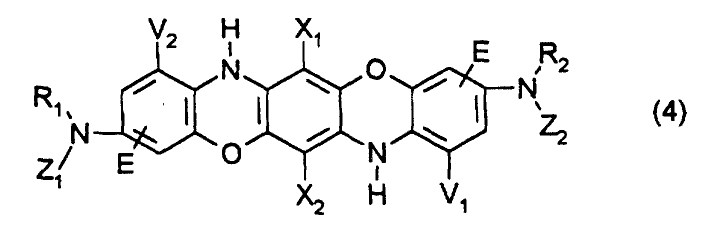 und diese anschließend zur Triphendioxazin-Verbindung der Formel (1) oxidiert.

2. Verfahren nach Anspruch 1, **dadurch gekennzeichnet, daß** die Reduktion in wäßrigem Medium durchgeführt wird.

3. Verfahren nach Anspruch 1 oder 2, **dadurch gekennzeichnet, daß** die Reste Z₁ und Z₂ für C₁-C₆-Alkyl-carbonyl, C₂-C₄-Alkenyl-carbonyl, C₆-Aryl-carbonyl, Aminocarbonyl, C₁-C₆-Alkylaminocarbonyl, Di-(C₁-C₆)-alkylaminocarbonyl, C₆-Arylamino-carbonyl oder C₆-Aryl-sulfonyl, wobei die Reste Alkyl und Aryl durch 1 bis 3, bevorzugt 1, gleiche oder verschiedene Substituenten aus der Reihe SO₂Y, Sulfo, Carboxy, Hydroxy, C₁-C₄-Alkoxy, C₁-C₄-Alkyl, Cyano, Halogen, Acylamino und Nitro substituiert sein können; oder für Reste der Formeln (5a) bis (5d) stehen, 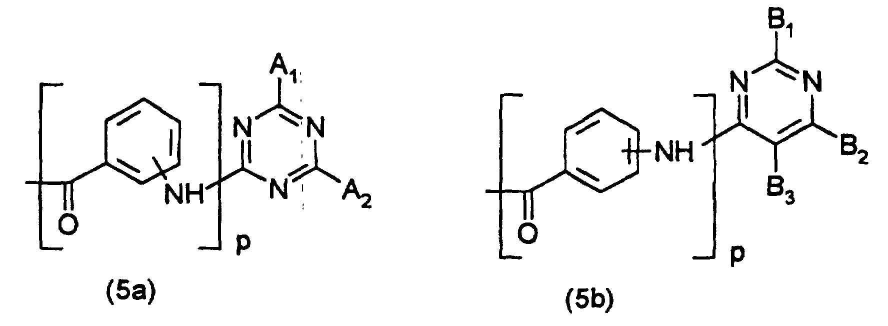 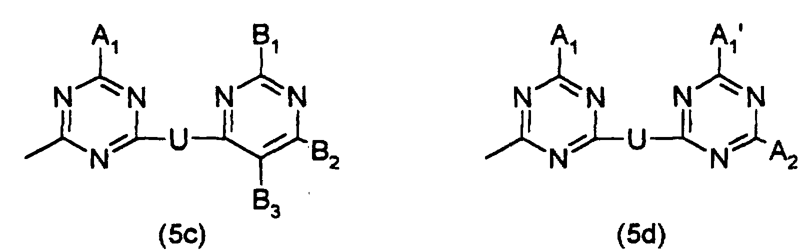 worin
p 0 oder 1, bevorzugt 0, ist;
A₁ Chlor, Fluor, C₁-C₄-Alkoxy, Phenoxy, Amino, Hydroxy, Cyanamino oder gegebenenfalls durch Carboxy oder Aminocarbonyl substituiertes Pyridinyl, oder ein Rest NR₇R₈ ist, worin
R₇ Wasserstoff, C₁-C₄-Alkyl, das durch 1 bis 2, bevorzugt 1, gleiche oder verschiedene Substituenten aus der Reihe Hydroxy, Sulfo, Sulfato und Carboxy substituiert sein kann,
oder Phenyl ist, das durch 1 bis 3 gleiche oder verschiedene Substituenten aus der Reihe Methoxy, Methyl, Halogen, Sulfo und Carboxy substituiert sein kann,
R₈ Wasserstoff oder C₁-C₄-Alkyl ist, das durch 1 bis 2, bevorzugt 1, Substituenten aus der Reihe Hydroxy, Sulfo, Sulfato und Carboxy substituiert sein kann,
oder R₇ und R₈ zusammen mit dem N-Atom einen gesättigten 5- bis 7-gliedrigen Heterocyclus bilden, der noch 1 bis 2 weitere der Heteroatome N, O und/oder S enthalten kann;
A₂ eine der Bedeutungen von A₁ hat;
A₁' eine der Bedeutungen von A₁ hat;
U ein Brückenglied aus der Reihe -NH-C₁-C₆-Alkylen-NH-, -NH-C₆-Arylen-NH-, wobei Arylen durch 1 bis 2 Reste Sulfo, Carboxy, Methyl und/oder Methoxy substituiert sein kann,
-NH-(C₆-C₁₀)-Aryl-(C₁-C₆)-alkylen-NH-, 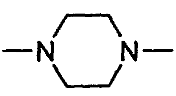 und 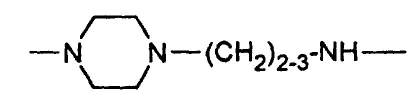 ist;
B₁ Wasserstoff, Chlor, Fluor, Trifluormethyl, Trichlormethyl oder Methylsulfonyl ist;
B₂ Wasserstoff, Chlor, Methyl, Methylsulfonyl oder Fluor ist;
B₃ Wasserstoff, Cyano, Fluor oder Chlor ist, mit der Maßgabe, daß
wenigstens einer der Reste B₁ oder B₂ eine Abgangsgruppe aus der Reihe Chlor, Fluor oder Methylsulfonyl ist;
oder für den Rest der Formel (5e) stehen 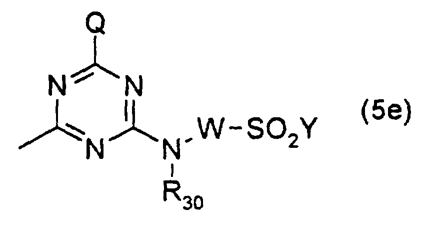 worin
Q Halogen, Hydroxy, Cyanamino oder ein Rest NR₃₀-W-SO₂Y ist,
W C₂-C₆-Alkylen, das durch eine Heterogruppe O, S, NH oder SO₂ unterbrochen sein kann, Phenylen, das durch Methoxy oder Sulfo substituiert sein kann, oder Aralkylen ist;
R₃₀ Wasserstoff, C₁-C₄-Alkyl, Phenyl, das durch eine Sulfogruppe substituiert sein kann, oder der Rest -W₁-SO₂Y ist, worin W₁ C₂-C₆-Alkylen ist, und
Y eine der in Anspruch 1 genannten Bedeutungen hat.

4. Verfahren nach mindestens einem der Ansprüche 1 bis 3, **dadurch gekennzeichnet, daß** Z₁ und Z₂ gleich oder verschieden sind und für Reste der Formeln 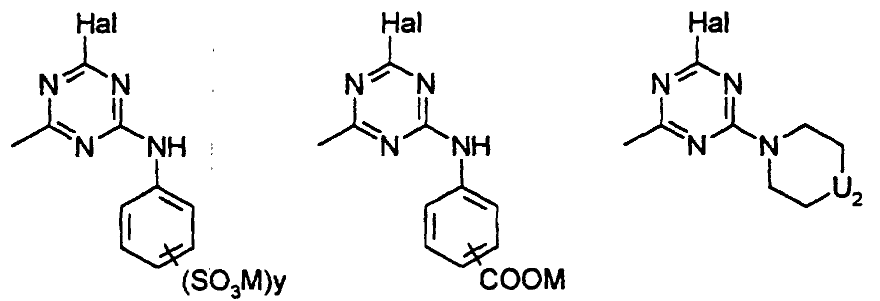 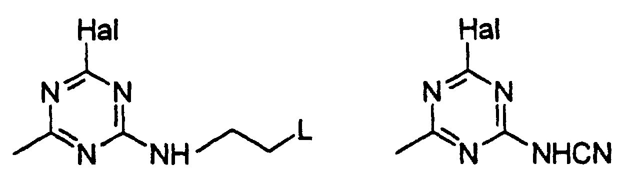 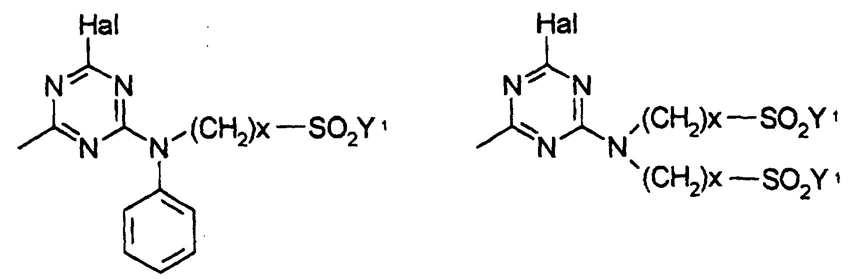 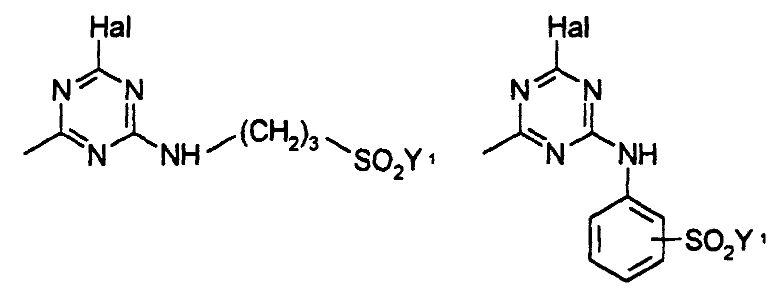 stehen, in welchen
x 2 oder 3;
y 1 oder 2;
U₂ eine chemische Bindung, Methylen, -O-, -NH-, >N(CH₂)₂OH, -S- oder -SO₂-;
L Hydroxy, Sulfo, Carboxy oder Sulfato;
Y¹ Vinyl, β-Sulfatoethyl, β-Chloroethyl oder β-Hydroxyethyl;
Hal Chlor oder Fluor, und
M Wasserstoff oder Alkalimetall ist.

5. Verfahren nach mindestens einem der Ansprüche 1 bis 4, wobei Z₁ und Z₂ gleich sind, **dadurch gekennzeichnet, daß** man die Verbindung der Formel (3), worin T die Bedeutung von Wasserstoff hat, mit einer 2- bis 6-fach molaren, vorzugsweise einer 2- bis 3-fach molaren, Menge des dem Rest Z₁ oder Z₂ zugrundeliegenden reaktiven Derivats umsetzt.

6. Verfahren nach mindestens einem der Ansprüche 1 bis 4, wobei Z₁ und Z₂ verschieden sind, **dadurch gekennzeichnet, daß** man die Verbindung der Formel (3), worin T die Bedeutung von Z₁ hat, mit einer 1- bis 3-fach molaren, vorzugsweise 1- bis 2-fach molaren, Menge des dem Rest Z₂ zugrundeliegenden reaktiven Derivats umsetzt.

7. Verfahren nach mindestens einem der Ansprüche 1 bis 6, **dadurch gekennzeichnet, daß** das den Resten Z₁ und Z₂ zugrundeliegende reaktive Derivat ein organisches oder anorganisches Säurehalogenid oder Anhydrid, ein Isocyanat, ein Kohlensäureester, Harnstoff, ein Carbaminsäurechlorid, ein Halogentriazin oder ein Halogenpyrimidin ist.

8. Verbindung der Formel (3) 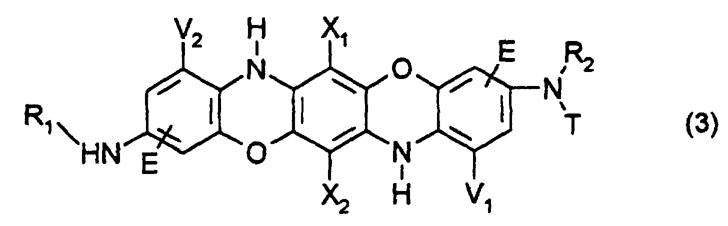 worin R₁, R₂, X₁, X₂, E, V₁, V₂ und T wie in Anspruch 1 definiert sind.

9. Verbindung der Formel (4) 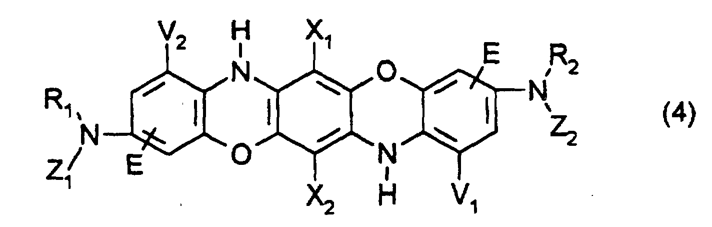 worin R₁, R₂, X₁, X₂, E, V₁, V₂, Z₁ und Z₂ wie in Anspruch 1 definiert sind.

## Claims

1. A process for the preparation of a triphendioxazine compound of the formula (1) 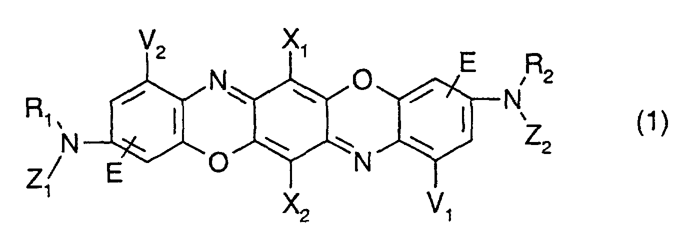 in which
R₁ is hydrogen or C₁-C₄-alkyl, which can be substituted by 1 or 2 substituents from the series consisting of hydroxyl, C₁-C₄-alkoxy, sulfato or sulfo;
R₂ has one of the meanings of R₁;
E is sulfo, carboxyl, C₁-C₄-alkylsulfonyl or a radical SO₂Y,
in which Y is vinyl or CH₂CH₂V, in which V is hydroxyl, or is a leaving group from the series consisting of sulfato, phosphato, thiosulfato or halogen;
or is -SO₂NR₃R₄ or -CONR₃R₄, in which
R₃ is hydrogen, phenyl, or C₁-C₄-alkyl, which can be substituted by hydroxyl, carboxyl, sulfo, sulfato or a radical SO₂Y,
R₄ has one of the meanings of R₃, or, together with R₃ and N, forms a 5- or 6-membered heterocyclic radical, which can be interrupted by 1 to 3 further heteroatoms from the series consisting of N, O and S;
X₁ is halogen, hydrogen, C₁-C₆-alkyl, phenyl, phenoxy or C₁-C₄-alkoxy;
X₂ has one of the meanings of X₁;
V₁ is hydrogen, sulfo, methoxy, methyl or halogen;
V₂ has one of the meanings of V₁; and
Z₁ and Z₂ are identical or different and are an acyl radical, an unsubstituted, alkylated or arylated aminocarbonyl radical, a
sulfonyl radical or a nitrogen-containing heteroaromatic radical,
which comprises reducing a compound of the formula (2) 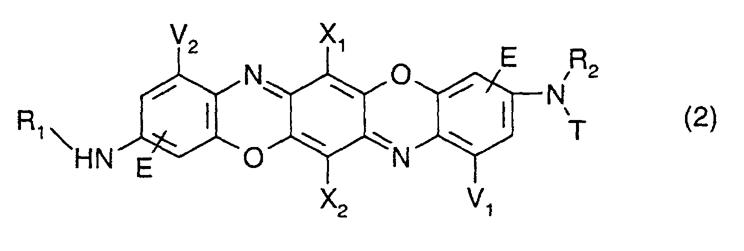 in which T is hydrogen, Z₁ or Z₂,
to give a compound of the formula (3), 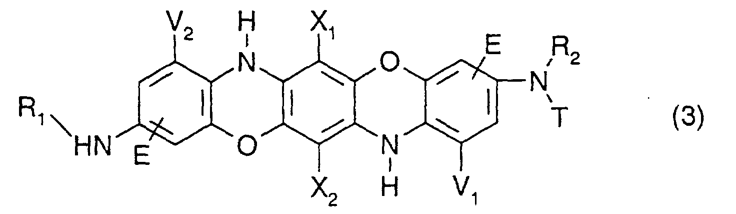 reacting this with a reactive derivative on which the radical Z₁ and/or Z₂ is based to give a compound of the formula (4) 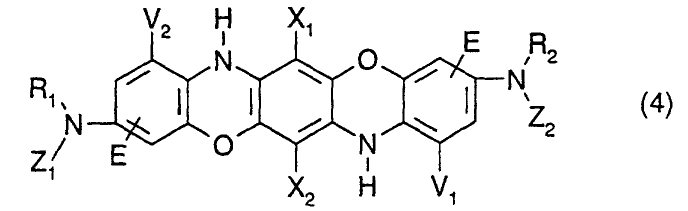 and subsequently oxidizing this to give the triphendioxazine compound of the formula (1).

2. The process as claimed in claim 1, wherein the reduction is carried out in an aqueous medium.

3. The process as claimed in claim 1 or 2, wherein the radicals Z₁ and Z₂ are C₁-C₆-alkyl-carbonyl, C₂-C₄-alkenylcarbonyl, C₆-aryl-carbonyl, aminocarbonyl, C₁-C₆-alkylaminocarbonyl, di-(C₁-C₆)-alkylaminocarbonyl, C₆-arylaminocarbonyl or C₆-aryl-sulfonyl, in which the alkyl and aryl radicals can be substituted by 1 to 3, preferably 1, identical or different substituents from the series consisting of SO₂Y, sulfo, carboxyl, hydroxyl, C₁-C₄-alkoxy, C₁-C₄-alkyl, cyano, halogen, acylamino and nitro; or are radicals of the formulae (5a) to (5d) 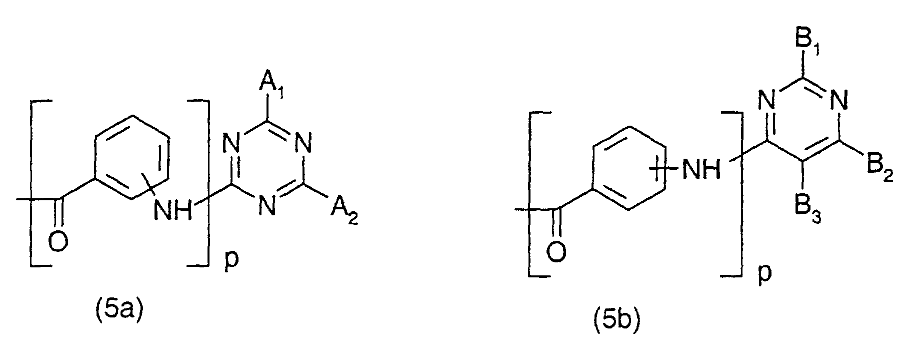 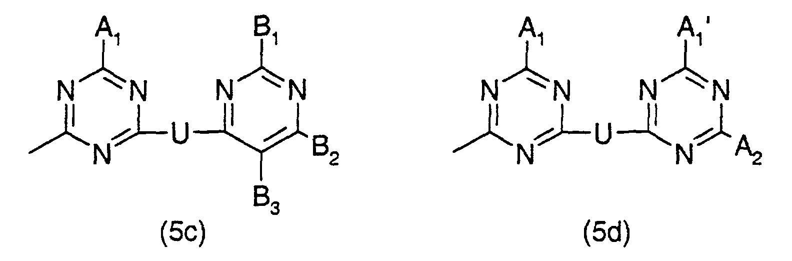 in which
p is 0 or 1, preferably 0;
A₁ is chlorine, fluorine, C₁-C₄-alkoxy, phenoxy, amino, hydroxyl, cyanoamino or pyridinyl which is optionally substituted by carboxyl or aminocarbonyl, or a radical NR₇R₈, in which
R₇ is hydrogen, C₁-C₄-alkyl, which can be substituted by 1 or 2, preferably 1, identical or different substituents from the series consisting of hydroxyl, sulfo, sulfato and carboxyl,
or phenyl, which can be substituted by 1 to 3 identical or different substituents from the series consisting of methoxy, methyl, halogen, sulfo and carboxyl,
R₈ is hydrogen or C₁-C₄-alkyl, which can be substituted by 1 or 2, preferably 1, substituents from the series consisting of hydroxyl, sulfo, sulfato and carboxyl,
or R₇ and R₈, together with the N atom, form a saturated 5- to 7-membered heterocyclic radical, which can also contain 1 or 2 others of the hetero groups N, O, and/or S;
A₂ has one of the meanings of A₁;
A₁' has one of the meanings of A₁;
U is a bridge member from the series consisting of -NH-C₁-C₆-alkylene-NH-, -NH-C₆-arylene-NH-, in which arylene can be substituted by 1 or 2 sulfo, carboxyl, methyl and/or methoxy radicals,
-NH-(C₆ -C₁₀)-aryl-(C₁-C₆)-alkylene-NH-, 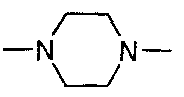 and 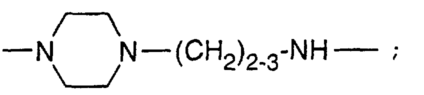
B₁ is hydrogen, chlorine, fluorine, trifluoromethyl, trichloromethyl or methylsulfonyl;
B₂ is hydrogen, chlorine, methyl, methylsulfonyl or fluorine; and
B₃ is hydrogen, cyano, fluorine or chlorine,
with the proviso that at least one of the radicals B₁ or B₂ is a leaving group from the series consisting of chlorine, fluorine and methylsulfonyl;
or are the radical of the formula (5e) 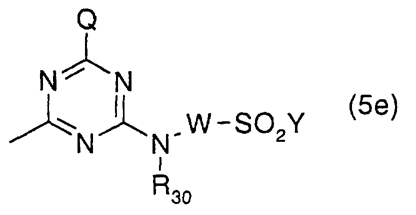 in which
Q is halogen, hydroxyl, cyanoamino or a radical NR₃₀-W-SO₂Y;
W is C₂-C₆-alkylene, which can be interrupted by a hetero group O, S, NH or SO₂, phenylene, which can be substituted by methoxy or sulfo, or aralkylene;
R₃₀ is hydrogen, C₁-C₄-alkyl, phenyl, which can be substituted by a sulfo group, or the radical -W₁-SO₂Y, in which W₁ is C₂-C₆-alkyl, and
Y has one of the meanings given in claim 1.

4. The process as claimed in at least one of claims 1 to 3, wherein Z₁ and Z₂ are identical or different and are radicals of the formulae 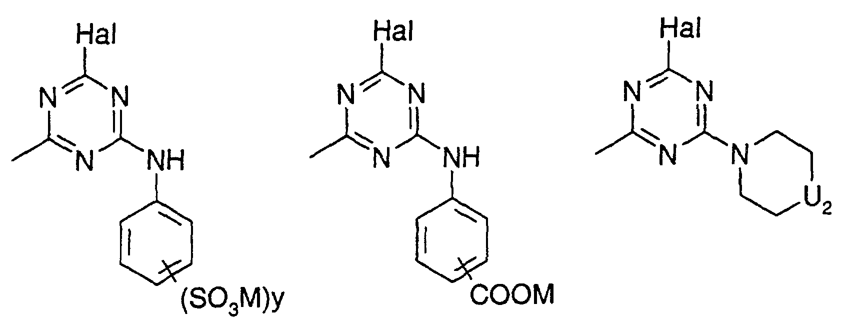 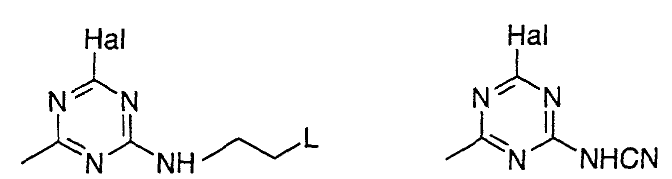 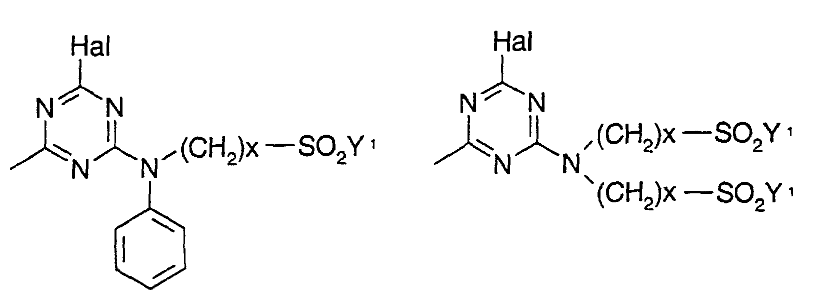 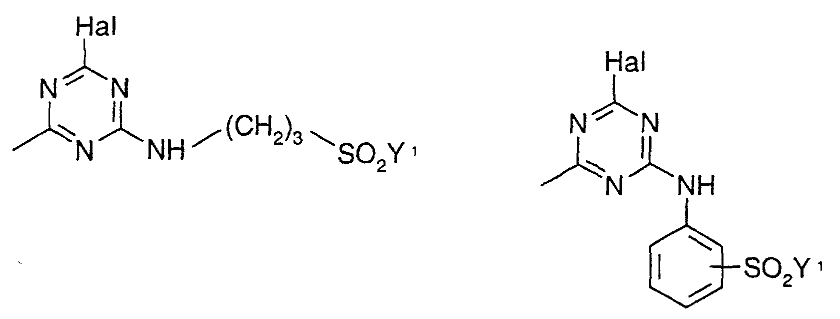 in which
x is 2 or 3;
y is 1 or 2;
U₂ is a chemical bond, methylene, -O-, -NH-, >N(CH₂)₂OH, -S- or -SO₂-;
L is hydroxyl, sulfo, carboxyl or sulfato;
Y¹ is vinyl, β-sulfatoethyl, β-chloroethyl or β-hydroxyethyl;
Hal is chlorine or fluorine, and
M is hydrogen or an alkali metal.

5. The process as claimed in at least one of claims 1 to 4, where Z₁ and Z₂ are identical, wherein the compound of the formula (3) in which T has the meaning of hydrogen is reacted with 2 to 6 times the molar amount, preferably 2 to 3 times the molar amount, of the reactive derivative on which the radical Z₁ or Z₂ is based.

6. The process as claimed in at least one of claims 1 to 4, wherein Z₁ and Z₂ are different, wherein the compound of the formula (3) in which T has the meaning of Z₁ is reacted with 1 to 3 times the molar amount, preferably 1 to 2 times the molar amount, of the reactive derivative on which the radical Z₂ is based.

7. The process as claimed in at least one of claims 1 to 6, wherein the reactive derivative on which the radicals Z₁ and Z₂ are based is an organic or inorganic acid halide or anhydride, an isocyanate, a carbonic acid ester, urea, a carbamic acid chloride, a halotriazine or a halopyrimidine.

8. A compound of the formula (3) 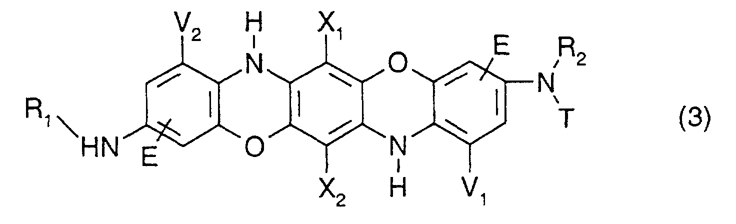 in which R₁, R₂, X₁, X₂, E, V₁, V₂ and T are defined as in claim 1.

9. A compound of the formula (4) 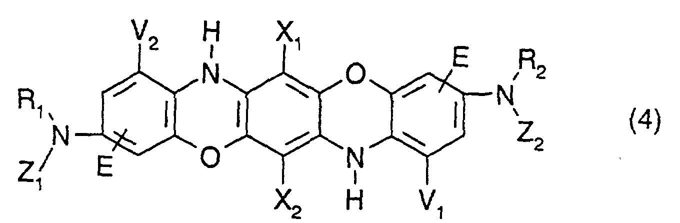 in which R₁, R₂, X₁, X₂, E, V₁, V₂, Z₁ and Z₂ are defined as in claim 1.

## Revendications

1. Procédé de préparation de composés triphénodioxazines de formule générale (1) 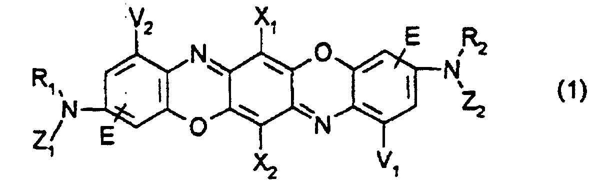 dans laquelle
R₁ représente un hydrogène ou un alkyle en C₁-C₄ qui peut être substitué par 1 à 2 substituants parmi le groupe constitué d'un hydroxy, d'un alcoxy en C₁-C₄, d'un sulfato ou d'un suifo ;
R₂ a l'une des significations de R₁ ;
E représente un sulfo, un carboxy, un C₁-C₄-alkylsulfonyle ou un radical SO₂Y, dans lequel Y représente un vinyle ou CH₂CH₂V, où V désigne un hydroxy ou un groupe partant parmi le groupe constitué d'un sulfato, d'un phosphato, d'un thiosulfato ou d'un halogène ;
ou représente -SO₂NR₃R₄ ou -CONR₃R₄, où
R₃ représente un hydrogène, un phényle ou un alkyle en C₁-C₄ qui peut être substitué par un hydroxy, un carboxy, un sulfo, un sulfato ou un radical SO₂Y,
R₄ a l'une des significations de R₃ ou forme, conjointement avec R₃ et N, un hétérocycle à 5 ou 6 chaînons qui peut être interrompu par 1 à 3 autres hétéro-atomes parmi le groupe constitué de N, O et S ;
X₁ représente un halogène, un hydrogène, un alkyle en C₁-C₆, un phényle, un phénoxy ou un alcoxy en C₁ - C₄ ;
X₂ a l'une des significations de X₁ ;
V₁ représente un hydrogène, un sulfo, un méthoxy, un méthyle ou un halogène ;
V₂ a l'une des significations de V₁; et
Z₁ et Z₂ sont identiques ou différents et représentent un radical acyle, un radical aminocarbonyle non substitué, alkylé ou arylé, un radical sulfonyle ou un radical hétéro-aromatique azoté,
**caractérisé en ce qu'**un composé de formule (2) 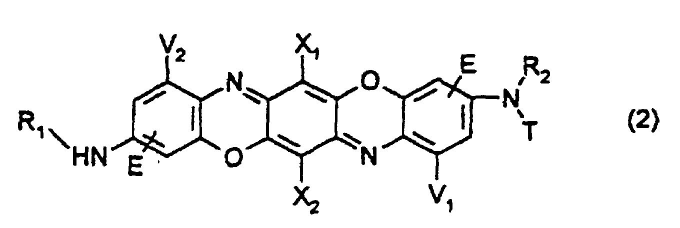 dans laquelle T représente un hydrogène, Z₁ ou Z₂, est réduit en un composé de formule (3), 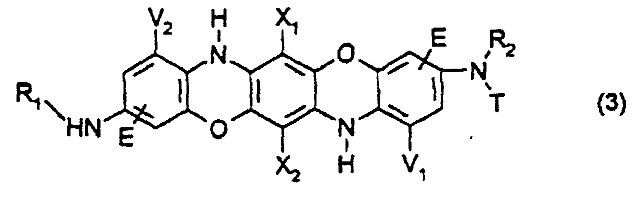 celui-ci est mis à réagir avec un dérivé réactif à la base du radical Z₁ et/ou Z₂ pour donner lieu à un composé de formule (4) 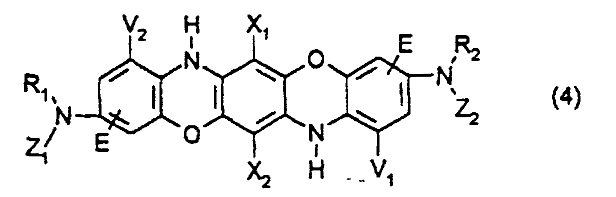 et celui-ci est ensuite oxydé pour donner le composé triphénodioxazine de formule (1).

2. Procédé selon la revendication 1, **caractérisé en ce que** la réduction est réalisée en milieu aqueux.

3. Procédé selon la revendication 1 ou 2, **caractérisé en ce que** les radicaux Z₁ et Z₂ désignent un C₁-C₆-alkylcarbonyle, un C₂-C₄-alcénylcarbonyle, un C₆-arylcarbonyle, un aminocarbonyle, un C₁-C₆-alkylaminocarbonyle, un di-(C₁-C₆)-alkylaminocarbonyle, un C₆-arylaminocarbonyle ou un C₆-arylsulfonyle, les radicaux alkyle et aryle pouvant être substitués par 1 à 3, de préférence 1, substituants identiques ou différents parmi le groupe constitué de SO₂Y, d'un sulfo, d'un carboxy, d'un hydroxy, d'un alcoxy en C₁-C₄, d'un alkyle en C₁-C₄, d'un cyano, d'un halogène, d'un acylamino et d'un nitro ; ou des radicaux de formules (5a) à (5d), 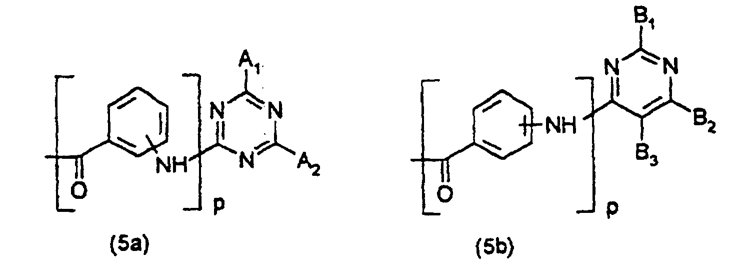 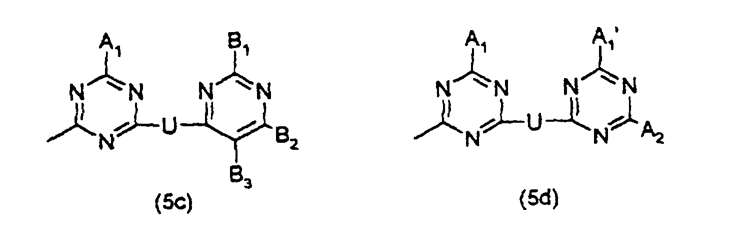 dans lesquelles
p vaut 0 ou 1, de préférence 0 ;
A₁ représente un chlore, un fluor, un alcoxy en C₁-C₄, un phénoxy, un amino, un hydroxy, un cyanoamino ou un pyridinyle éventuellement substitué par un carboxy ou un aminocarbonyle, ou un radical NR₇-R₈ dans lequel
R₇ représente un hydrogène, un alkyle en C₁-C₄, qui peut être substitué par 1 à 2, de préférence 1, substituants identiques ou différents parmi le groupe constitué d'un hydroxy, d'un sulfo, d'un sulfato et d'un carboxy, ou un phényle qui peut être substitué par 1 à 3 substituants identiques ou différents parmi le groupe constitué d'un méthoxy, d'un méthyle, d'un halogène, d'un sulfo et d'un carboxy,
R₈ représente un hydrogène ou un alkyle en C₁-C₄ qui peut être substitué par 1 à 2, de préférence 1 substituants parmi le groupe constitué d'un hydroxy, d'un sulfo, d'un sulfato et d'un carboxy,
ou R₇ et R₈ forment, conjointement avec l'atome d'azote, un hétérocycle saturé à 5 à 7 chaînons qui peut également renfermer de 1 à 2 autres hétéro-atomes parmi N, O et/ou S ;
A₂ a l'une des significations de A₁ ;
A₁' a l'une des significations de A₁ ;
U représente un groupe de pontage parmi le groupe constitué de -NH-C₁-C₆-alkylène-NH-, -NH-C₆-arylène-NH-, où l'arylène peut être substitué par 1 à 2 radicaux sulfo, carboxy, méthyl et/ou méthoxy,
-NH-(C₆-C₁₀)-Aryl-(C₁-C₆)alkylène-NH-, 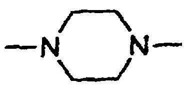 et 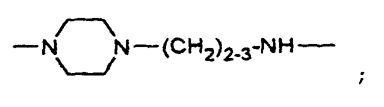
B₁ représente un hydrogène, un chlore, un fluor, un trifluorométhyle, un trichlorométhyle ou un méthylsufonyle ;
B₂ représente un hydrogène, un chlore, un méthyle, un méthylsulfonyle ou un fluor ;
B₃ représente un hydrogène, un cyano, un fluor ou un chlore, à condition qu'au moins l'un des radicaux B₁ ou B₂ soit un groupe partant parmi le groupe constitué d'un chlore, d'un fluor ou d'un méthylsulfonyle ;
ou désignent le radical de formule (5e) 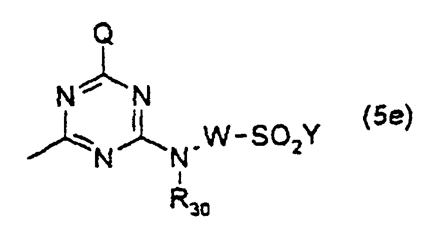 dans laquelle
Q représente un halogène, un hydroxy, un cyanamino ou un radical NR₃₀-W-SO₂Y,
W représente un alkylène en C₂-C₆ qui peut être interrompu par un hétérogroupe O, S, NH ou SO₂, un phénylène qui peut être substitué par un méthoxy ou un sulfo, ou un aralkylène ;
R₃₀ représente un hydrogène, un alkyle en C₁-C₄, un phényle qui peut être substitué par un groupe sulfo, ou le radical -W₁-SO₂Y dans lequel W₁ représente un alkylène en C₂-C₆, et
Y a l'une des significations mentionnées à la revendication 1.

4. Procédé selon au moins l'une des revendications 1 à 3, **caractérisé en ce que** Z₁ et Z₂ sont identiques ou différents et désignent des radicaux de formules 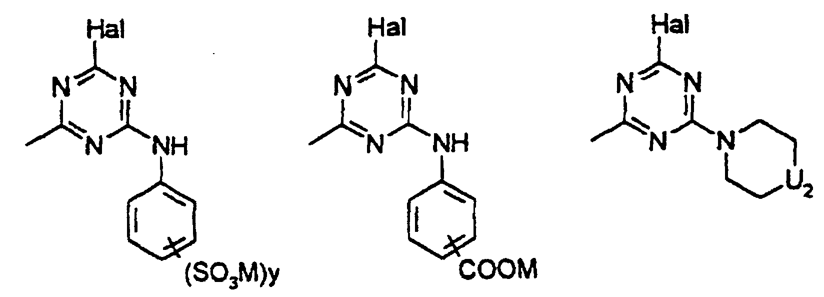 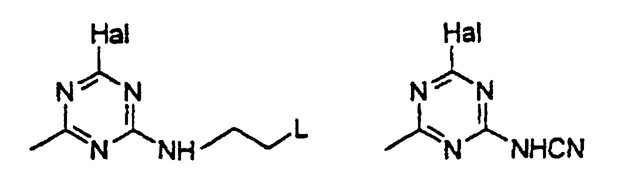 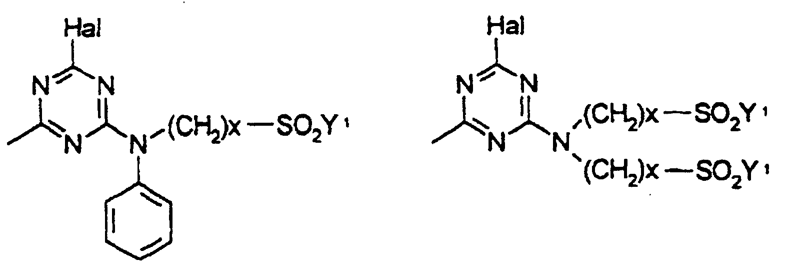 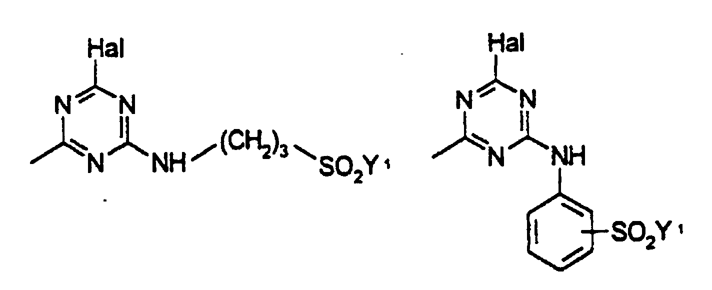 dans lesquelles
x vaut 2 ou 3 ;
y vaut 1 ou 2 ;
U₂ représente une liaison chimique, un méthylène, -O-, -NH-, >N(CH₂)₂OH, -S- ou -SO₂- ;
L représente un hydroxy, un sulfo, un carboxy ou un sulfato ;
Y¹ représente un vinyle, un β-sulfatoéthyle, un β-chloroéthyle ou un β-hydroxyéthyle ;
Hal représente un chlore ou un fluor, et
M représente un hydrogène ou un métal alcalin.

5. Procédé selon au moins l'une des revendications 1 à 4, dans lequel Z₁ et Z₂ sont identiques, **caractérisé en ce que** le composé de formule (3) dans laquelle T représente un hydrogène, est mis à réagir avec une quantité molaire 2 à 6 fois supérieure, de préférence 2 à 3 fois supérieure, du dérivé réactif à la base du radical Z₁ ou Z₂.

6. Procédé selon au moins l'une des revendications 1 à 4, dans lequel Z₁ et Z₂ sont différents, **caractérisé en ce que** le composé de formule (3) dans laquelle T a la signification de Z₁, est mis à réagir avec une quantité molaire 1 à 3 fois supérieure, de préférence 1 à 2 fois supérieure, du dérivé réactif à la base du radical Z₂.

7. Procédé selon au moins l'une des revendications 1 à 6, **caractérisé en ce que** le dérivé réactif à la base des radicaux Z₁ et Z₂ est un anhydride ou halogénure d'acide organique ou inorganique, un isocyanate, un ester carbonique, l'urée, un chlorure d'acide carbamique, une halogénotriazine ou une halogénopyrimidine.

8. Composé de formule (3) 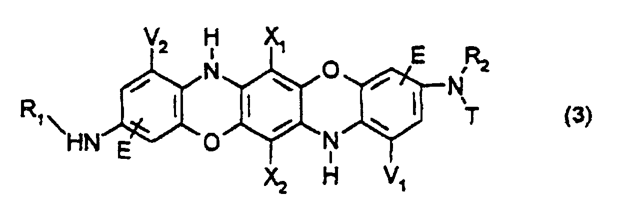 dans laquelle R₁, R₂, X₁, X₂, E, V₁, V₂ et T sont tels que définis à la revendication 1.

9. Composé de formule (4) 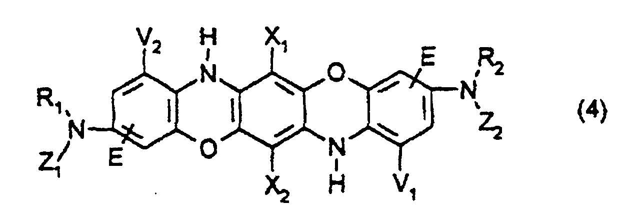 dans laquelle R₁, R₂, X₁, X₂, E, V₁, V₂, Z₁ et Z₂ sont tels que définis à la revendication 1.
